# EUROPEAN PATENT SPECIFICATION

(11) **EP 0 771 876 B1**
(45) Date of publication and mention of the grant of the patent: **10.07.2002**
(21) Application number: 96905023.6
(22) Date of filing: 07.03.1996
(51) Int. Cl.: C12N 15/53, C12N 1/19, C12N 9/06, C12P 13/24, C12N 9/02

(54) **PROCESS FOR PRODUCING cis-3-HYDROXY-L-PROLINE**
VERFAHREN ZUR HERSTELLUNG VON cis-3-HYDROXY-L-PROLIN
PROCEDE D'OBTENTION DE cis-3-HYDROXY-L-PROLINE

(30) Priority: 07.03.1995 JP 4698795
(43) Date of publication of application: 07.05.1997
(73) Proprietor: KYOWA HAKKO KOGYO CO., Ltd., Chiyoda-ku, Tokyo 100 (JP)
(72) Inventor: OZAKI, Akio, Machida-shi, Tokyo 194 (JP); MORI, Hideo, Machida-shi, Tokyo 194 (JP); SHIBASAKI, Takeshi, Kawasaki-shi, Kanagawa 215 (JP)
(74) Representative: Vossius, Volker, Dr.
(86) International application number: JP9600558
(87) International publication number: WO9627668

(56) References cited:
- EP-A- 0 645 457
- DATABASE WPI Week 9605 Derwent Publications Ltd., London, GB; AN 96-040697 XP002099047 & CA 2 131 776 A (KYOWA HAKKO KOGYO KK) , 6 October 1995

## Description

### Field of the Invention

The present invention relates to industrial methods for producing cis-3-hydroxy-L-proline which is useful as a starting compound for medicines and an additive to foods, genes which code for a protein having the enzymatic activity of hydroxylating the 3-position of L-proline and which are useful for the above-mentioned process (hereinafter referred to as L-proline 3-hydroxylase genes), transformants containing the gene, and methods for producing L-proline 3-hydroxylases using the transformants.

### Background of the Invention

Heretofore, chemosynthetic methods of producing cis-3-hydroxy-L-proline are known [J. Amer. Chem. Soc., 84, 3980 (1962); J. Amer. Chem. Soc., 85, 2824 (1963); Nature 289, 310 (1981); J. Org. Chem., 54, 1866 (1989); Acta Chemica Scandinavica, 43, 290 (1989)].

No chemosynthetic or biological method of producing cis-3-hydroxy-L-proline by hydroxylating L-proline both regio-selectively and stereo-selectively, had been reported yet.

The conventional chemosynthetic methods for producing cis-3-hydroxy-L-proline are not satisfactory for industrial production, because of (1) the expensive raw materials, (2) too many the reaction steps, (3) the complicated procedures for isolating and purifying the product and/or (4) the lower productivity of cis-3-hydroxy-L-proline.

Industrial methods for advantageously producing cis-3-hydroxy-L-proline by using L-proline 3-hydroxylases with high activity are desired.

The object of the present invention is to provide methods for efficiently producing cis-3-hydroxy-L-proline from inexpensive and easily-available L-proline by using L-proline 3-hydroxylases, for which, in order to more industrially advantageously produce cis-3-hydroxy-L-proline, L-proline 3-hydroxylase genes and transformants containing the gene are provided. L-proline 3-hydroxylases are produced in large quantities by using the genes and the transformants, and cis-3-hydroxy-L-proline is industrially produced at low costs by using the transformants or the hydroxylases.

### DISCLOSURE OF THE INVENTION

The present invention relates to novel, microorganism-derived L-proline 3-hydroxylase genes, to recombinant DNAs containing the gene, to transformants containing the recombinant DNA, to methods for producing L-proline 3-hydroxylases using the transformants, to the hydroxylases, and to methods for producing cis-3-hydroxy-L-proline using the transformants or the hydroxylases

The L-proline-3-hydroxylases of the present invention are enzymes which hydroxylate free L-proline in the presence of 2-ketoglutaric acid and divalent iron ions to produce cis-3-hydroxy-L-proline.

The present invention encompasses any and every protein having the enzymatic activity of hydroxylating the 3-position of L-proline, which includes, for example, a protein having the amino acid sequence indicated by Sequence No. 1 or 2, a fused protein having an amino acid sequence that results from the protein or a protein having a partial amino acid sequence of the protein as bonded to a peptide having a partial amino acid sequence of an Escherichia coli-derived β-galactosidase protein, a fused protein having an amino acid sequence that results from the protein having the amino acid sequence indicated by Sequence No. 1 or 2 or a protein having a partial amino acid sequence of the protein as bonded to a peptide having a partial amino acid sequence of an E. coli-derived maltose-binding protein, etc. Examples of the fused proteins include proteins having the amino acid sequence as indicated by Sequence No. 15, 16 or 17, etc.

The amino acid sequence indicated by Sequence No. 1, 2, 15, 16 or 17 includes proteins having an amino acid sequence with one or more amino acids substituted, deleted or added and having the enzymatic activity of hydroxylating the 3-position of L-proline. The substitution, the deletion and the addition of amino acids can be conducted in accordance with the methods described in Nucleic Acids Research, Vol. 10, pp. 6487-6500 (1982); Proc. Natl. Acad. Sci., USA, Vol. 79, pp. 6409-6413 (1982); Proc. Natl. Acad. Sci., USA, Vol.81, pp. 5662-5666 (1984); Science, Vol. 224, pp. 1431-1433 (1984); PCT WO85/00817 (1985); Nature, Vol. 316, pp. 601-605 (1985); Gene, Vol. 34, pp. 315-323 (1985); Nucleic Acids Research, Vol. 13, pp. 4431-4442 (1985); Current Protocols in Molecular Biology, Chap. 8, Mutagenesis of Cloned DNA, John Wiley & Sons, Inc. (1989), etc.

The present invention encompasses any and every L-proline-3-hydroxylase genes of a DNA fragment containing a gene that codes for a protein having the enzymatic activity of hydroxylating the 3-position of L-proline, and this may include, for example, genes coding for the protein having the amino acid sequence as indicated by Sequence No. 1, 2, 15, 16, or 17, and also genes which code for a protein that has an amino acid sequence corresponding to the amino acid sequence as indicated by Sequence No. 1, 2, 15, 16 or 17 and derived therefrom by substitution, deletion or addition of at least one amino acid and which have the enzymatic activity of hydroxylating the 3-position of L-proline. Concretely mentioned are DNAs indicated by Sequence Nos. 3, 4, 13 and 14.

The L-proline-3-hydroxylases genes of the present invention include the DNAs as defined hereinabove and also DNAs as derived therefrom by mutation, such as substituting mutation, deleting mutation, inserting mutation or the like, to be conducted to the extent that the mutated DNAs do not lose the L-proline-3-hydroxylases activity, for example, DNAs with homology to Sequence No. 3, 4, 13 or 14. Such homologous DNAs are those to be obtained by colony hybridization or plaque hybridization using, as a probe, the DNA having the nucleotide sequence as indicated by Sequence No. 3, 4, 13 or 14. These treatments can be conducted in accordance with known in vitro recombination techniques [see Molecular Cloning: A Laboratory Manual, 2nd Ed., edited by Sambrook, Fritsch, Maniatis, published by Cold Spring Harbor Laboratory Press, 1989].

DNA fragments containing the L-proline-3-hydroxylases gene can be obtained from microorganisms having the ability of hydroxylating L-proline to produce cis-3-hydroxy-L-proline. As the microorganisms, any microorganism having the ability of hydroxylating L-proline to produce cis-3-hydroxy-L-proline can be employed in the present invention. As preferable example of such a microorganism, microorganisms belonging to the genus Streptomyces or Bacillus and having the enzymatic activity of hydroxylating the 3-position of L-proline can be mentioned. More preferable examples thereof include Streptomyces canus ATCC12647, Streptomyces canus ATCC12646, Streptomyces sp. TH1 (FERM BP-4399), Bacillus sp. TH2 (FERM BP-4397), Bacillus sp. TH3 (FERM BP-4398), or mutants or derivatives of these strains.

Methods for obtaining L-proline-3-hydroxylases genes derived from microorganisms having the ability of producing L-proline-3-hydroxylases are described below.

Chromosomal DNA is prepared from a microorganism having the ability of producing L-proline-3-hydroxylase through a usual DNA isolation method, for example, a phenol method [see Biochim. Biophys. Acta, 72, 619-629 (1963)]. The thus-obtained chromosomal DNA is cleaved with suitable restriction enzymes, then the resulting fragments are inserted into vector DNAs to construct chromosomal DNA libraries for the chromosomes of the microorganisms. Using the chromosomal DNA library, host microorganisms can be transformed. The transformants containing the L-proline-3-hydroxylase gene are selected from the obtained transformants by the hybridization method. DNAs containing the intended gene can be obtained from the thus-selected transformants.

The process comprising a series of such steps can be conducted in accordance with known in vitro recombination method (molecular Cloning, A Laboratory Manual, 2nd edition, edited by Sambrook, Fritsch and Maniatis, Cold Spring Harbor Laboratory Press, 1989).

As the vector DNAs that are used to construct the chromosomal DNA library of the microorganism having the ability of producing L-proline-3-hydroxylase, phage vectors and plasmid vectors can be used if they can be replicated autonomously in Escherichia coli K12 strain. Preferable examples of the vector DNA include λ ZAPII, pUC18 and pBluescript (commercially available from STRATAGENE Co.).

As the host microorganisms that are used to construct the chromosomal DNA library of the microorganism having the ability of producing L-proline-3-hydroxylase, any of the microorganisms belonging to the genus Escherichia can be used. Preferable examples of the host microorganisms include E. coli XL1-Blue, E. coli XL2-Blue, E. coli DH1, E. coli MC1000, etc.

Based on the information about the amino acid sequence of L-proline-3-hydroxylase, DNA primers are synthesized. Using the DNA primers, DNA fragments are prepared through polymerase chain reaction (hereinafter referred to as PCR). Using the thus-obtained DNA fragments, transformants containing an L-proline-3-hydroxylase gene can be selected by the hybridization method.

The information on the amino acid sequences of L-proline-3-hydroxylases can be obtained through analysis of pure L-proline-3-hydroxylases using ordinary amino acid sequencers, such as Protein Sequencer Model PPSQ-10 (produced by Shimadzu Seisakusho K.K.). As the information on the amino acid sequences thus obtained, concretely mentioned are the amino acid sequences as indicated by Sequence Nos. 5 to 7, etc.

The DNA primers can be synthesized by means of an ordinary DNA synthesizers, for example, 380A·DNA Synthesizer (produced by Applied Biosystems Co.), etc.

As the probes for the hybridization, usable are partial fragments of L-proline-3-hydroxylases genes, which can be obtained through PCR. For example, a DNA as indicated by Sequence No. 8 in the Sequence List (this corresponds to a sense chain DNA coding for from the first to the sixth amino acids in the amino acid sequence of Sequence No. 1) and a DNA as indicated by Sequence No. 10 in the Sequence List (this corresponds to an anti-sense chain DNA coding for from the 21st to 25th amino acids in the amino acid sequence of Sequence No. 1) are chemically synthesized. Through PCR using these partial fragment as DNA primers, obtained is a DNA fragment of 74 bp as indicated by Sequence No. 11. The thus-obtained DNA fragment can be used as the probe for the hybridization.

The DNA which is obtained from the transformant as selected through the hybridization and which contains an L-proline-3-hydroxylases gene is cleaved with suitable restriction enzymes, such as PstI or the like, and then cloned into plasmids, such as pBluescript KS(+) (commercially available from STRATAGENE Co.). The nucleotide sequence of the above-mentioned gene can be determined by ordinary base sequencing methods, such as the dideoxy chain termination method of Sanger et al[see Proc. Natl. Acad. Sci., USA, 74, 5463 (1977)]. The determination of the nucleotide sequence can be conducted using automatic DNA sequencers, such as 373A·DNA Sequencer (produced by Applied Biosystems Co.) or the like.

As the thus-determined nucleotide sequences of L-proline-3-hydroxylases genes, for example the nucleotide sequences as indicated by Sequence Nos. 3, 4, 13 and 14 can be mentioned.

As the plasmid containing the DNA that codes for an L-proline-3-hydroxylases of the present invention, for example, pTH30, pTH71, pTH75, etc. can be mentioned. Escherichia coli XL2-Blue/pTH30 which is an Escherichia coli strain containing pTH30 and Escherichia coli XL2-Blue/pTH75 which is an Escherichia coli strain containing pTH75 were deposited at the National Institute of Bioscience and Human-Technology of the Agency of Industrial Science and Technology (which is located at 1-3, Higashi 1-chome, Tsukuba-shi, Ibaraki-ken 305, Japan) as of March 2, 1995 under FERM BP-5026 for the strain XL2-Blue/pTH30 and as of February 22, 1996 under FERM BP-5409 for the strain XL2-Blue/pTH75, both in terms of the Budapest Treaty.

To express the thus-obtained L-proline-3-hydroxylase genes in hosts, the DNA fragment containing the L-proline-3-hydroxylase gene is first cleaved with a restriction enzyme or deoxyribonucleases (DNases) to form a DNA fragment of a suitable length containing the L-proline-3-hydroxylases gene. The thus-formed DNA fragment is inserted into an expression vector at the downstream position of the promoter therein, and thereafter the expression vector having the thus-inserted DNA therein is introduced into a host cell suitable for the expression vector.

As the host, usable is any one capable of being expressed in the intended gene. For example, the host includes microorganisms belonging to the genus Escherichia, Serratia, Corynebacterium, Brevibacterium, Pseudomonas, Bacillus, etc., as well as yeast strains, animal cell hosts, etc.

As the expression vector, usable is one which is autonomously replicable in the above-mentioned hosts or capable of being inserted into their chromosomes and which contains a promoter at the position at which the L-proline-3-hydroxylases gene can be transcribed.

When the microorganisms such as Escherichia coli or the like are used as the host, it is desirable that the L-proline-3-hydroxylases expression vector is replicable autonomously in the microorganisms and is composed of a promoter, a ribosome-binding sequence, an L-proline-3-hydroxylase gene and a transcription terminator sequence. A regulatory gene may be contained therein.

As examples of the expression vector, mentioned are pBTrp2, pBTac1, pBTac2 (all commercially available from Boehringer Mannheim Co.); pKYP10 (see Japanese Published Unexamined Patent Application No. 58-110600); pKYP200 [see Agric. Biol. Chem., Vol. 48, pp. 669-675 (1984)]; pLSA1 [see Agric. Biol. Chem., Vol. 53, p. 277 (1989)]; pGEL1 [see Proc. Natl. Acad. Sci., USA, Vol. 82, p. 4306 (1985)]; pBluescript (produced by STRATAGENE Co.); pTrs30 [prepared from Escherichia coli JM109/pTrS30 (FERM BP-5407); pTrs32 [prepared from Escherichia coli JM109/pTrs32 (FERM BP-5408)], etc.

As the promoter, usable is any one capable of being expressed in hosts such as Escherichia coli, etc. For example, mentioned are promoters derived from Escherichia coli, phage, etc., such as trp promoter (Ptrp), lac promoter (Plac), P_{L} promoter and P_{R} promoter. Also usable are artificially designed and modified promoters, such as Ptrpx2 to be prepared by connecting two Ptrp's in series, as well as tac promoter.

As the ribosome-binding sequence, any one capable of being expressed in hosts such as Escherichia coli can be used. However, it is desirable to use plasmids having a ribosome-binding sequence and an initiation codon as spaced at suitable intervals therebetween (for example, by from 6 to 18 bases).

The L-proline-3-hydroxylase gene may be any and every gene that codes for an L-proline-3-hydroxylase. However, it is desirable that the bases constituting the DNA sequence of the gene are suitably substituted in order that the substituted DNA sequence can be constituted of codon most suitable for expression in the host microorganisms to be used.

Transcription terminator sequences are not always necessary for the expression of the genes of the present invention. However, it is desirable that a transcription terminator sequence is arranged just after the structural gene.

Examples of the hosts usable in the present invention include Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Bacillus subtilis, Bacillus amyloliquefacines, Brevibacterium immariophilum ATCC14068, Brevibacterium saccharolyticum ATCC14066, Brevibacterium flavum ATCC14067, Brevibacterium lactofermentum ATCC13869, Corynebacterium glutamicum ATCC13032, Corynebacterium acetoacidophilum ATCC13870, Microbacterium ammoniaphilum ATCC15354, etc.

When yeast strains are used as the host, for example, YEp13 (ATCC37115), YEp24 (ATCC37051), YCp50 (ATCC37419), etc. can be used as the expression vector.

As the promoter, any one that can be expressed in the host of yeast strains can be used. For example, usable are promoters in genes of glycolases, such as hexosekinase, etc., as well as other promoters such as gal 1 promoter, gal 10 promoter, heat-shock protein promoters, MF α1 promoter, CUP 1 promoter, etc.

As examples of the host cells, Saccharomyces cerevisae, Schizosaccharomyces pombe, Kluyveromyces lactis, Trichosporon pullulans, Schwanniomyces alluvius, etc. can be mentioned.

When animal cells are used as the host, for example, pcDNA I/Amp, pcDNA I, pcDM8 (all commercially available from Funakoshi Co.), etc. can be used as the expression vector. As the promoter, any one that can be expressed in the host of animal cells can be used. For example, usable are promoters such as those in human CMV IE (immediate early) genes. An enhancer of human CMV IE genes can be used along with the promoter.

As examples of the host cells, Namalwa cells, HBT 5637 cells (see Japanese Published Unexamined Patent Application No. 299/88), COS cells, CHO cells, etc. can be used.

To introduce DNA into animal cells, any and every method capable of introducing DNA into animal cells can be employed herein. For example, employable are electroporation methods [see Miyaji et al., Cytotechnology, 3, 133 (1990)], calcium phosphate methods (see Japanese Published Unexamined Patent Application No. 227075/90), lipofection methods [see Philip L. Felgner, et al., Proc. Natl. Acad. Sci., USA, 84, 7413 (1987)], etc. The resulting transformants can be collected and cultivated in accordance with the methods described in Japanese Published Unexamined Patent Application Nos. 227075/90 and 257891/90.

The transformants obtained in the manner mentioned above can be cultivated according to ordinary incubation methods.

As the media for cultivating the transformants obtained by using the hosts of microorganisms such as Escherichia coli, yeast strains, etc., both natural and synthetic media can be employed so long as they properly contain carbon sources, nitrogen sources, inorganic salts and other materials capable of being assimilated by the microbial hosts and in which the transformants can be efficiently cultivated.

Any carbon sources that can be assimilated by the microorganisms may be used. Examples of the carbon source include carbohydrates such as glucose, fructose, sucrose, molasses containing these components, starch and starch hydrolyzates; organic acids such as acetic acid and propionic acid; and alcohols such as ethanol and propanol.

As the nitrogen sources, ammonia, ammonium salts of inorganic and organic acids such as ammonium chloride, ammonium sulfate, ammonium acetate and ammonium phosphate, other nitrogen-containing compounds, peptone, meat extracts, yeast extracts, corn steep liquor, casein hydrolyzates, soybean cakes, soybean cake hydrolyzates, cultured fermented cells, their digested products, etc. may be used.

As inorganic salts, potassium dihydrogen phosphate, dipotassium hydrogen phosphate, magnesium phosphate, magnesium sulfate, sodium chloride, ferrous sulfate, manganese sulfate, copper sulfate, calcium carbonate, etc. may be used.

The cultivation is conducted under aerobic conditions, for example, with shaking culture or submerged-aerial stirring culture. The temperature for the cultivation is 15 to 40°C. The period for the cultivation is usually 16 to 96 hours. During the cultivation, the pH of the medium is kept at 3.0 to 9.0. The pH is adjusted using inorganic or organic acids, alkaline solutions, urea, calcium carbonate, ammonia or the like.

L-Proline is suitably added to the media in such a manner that its concentration may be from 5 to 1000 mM, preferably from 20 to 200 mM, whereby the intended L-proline-3-hydroxylases can be produced more efficiently.

Antibiotics such as ampicillin, tetracycline or the like may be added to the medium during the cultivation, if required.

For the cultivation of the microorganisms which are transformed with the expression vector using the inducible promoter, inducers may be added to the medium, if required. For example, in cultivation of microorganisms transformed with the expression vector using lac promoter, isopropyl-β-D-thiogalactopyranoside (IPTG) may be added to the medium. In cultivation of microorganisms transformed with the expression vector using trp promoter, indoleacrylic acid (IAA) may be added to the medium.

As the medium for cultivating the transformants which are obtained by using the animal cells as a host cell, RPMI1640 medium and Eagle's MEM medium which are generally used or these culture media containing a fetal bovine serum can be used.

The cultivation of the cells is conducted in the presence of 5% CO₂. The temperature for the cultivation is preferably 35 to 37°C, and the period for the cultivation is usually 3 to 7 days.

L-Proline is suitably added to the media in such a manner that its concentration may be from 5 to 1000mM, preferably from 20 to 200 mM, whereby the intended L-proline-3-hydroxylases can be produced more efficiently.

Antibiotics such as kanamycin, penicillin or the like may be added to the medium during the cultivation, if required.

A considerable amount of L-proline-3-hydroxylase is produced and accumulated in the thus-cultivated transformants in comparison to the microorganism strain used as the gene source, such as Streptomyces sp. TH1 or the like. Thus, the isolation and purification of the enzyme or the production of cis-3-hydroxy-L-proline from L-proline using the enzyme can be performed far more efficiently in comparison to the production of cis-3-hydroxy-L-proline from L-proline using the non genetically-engineered microorganism as the enzyme source, such as Streptomyces sp. TH1 or the like.

The production of L-proline-3-hydroxylase in the transformants can be carried out by adding the culture, the cells or the treated cells to an aqueous medium suitable for the enzymatic reaction together with L-proline, a divalent iron ion and 2-ketoglutaric acid, and adding a surfactant or an organic solvent, if required, to determine cis-3-hydroxy-L-proline produced. With respect to the activity of the L-proline-3-hydroxylase of which the formation is confirmed in the cell, the activity of the enzyme for producing 1 nmol of cis-3-hydroxy-L-proline for 1 minute under the following conditions is defined as 1 unit (U). The microorganism cells and the animal cells are here called cells.

### Measurement of L-proline-3-hydroxylase activity:

The cells, the treated cells or the enzyme preparation are added to 200 mM TES [N-tris(hydroxymethyl)methyl-2-aminoethanesulfonic acid] buffer containing 12 mM L-proline, 24 mM 2-ketoglutaric acid, 4 mM ferrous sulfate and 8 mM L-ascorbic acid to make 250 µl in total. The mixture is kept at 35°C for 10 minutes. The reaction mixture is heated at 100°C for 2 minutes to stop the reaction, and the amount of cis-3-hydroxy-L-proline produced in the reaction mixture is determined by high-performance liquid chromatography (hereinafter referred to as HPLC).

To determine the amount of cis-3-hydroxy-L-proline formed, any method capable of determining it can be employed. For example, employable are a method of separating and eluting cis-3-hydroxy-L-proline from the reaction mixture by means of HPLC using a ligand exchange chromatography column, such as SUMICHIRAL OA5000 (produced by Sumika Analysis Center Co.), or the like, followed by reacting it with 7-chloro-4-nitrobenz-2-oxa-1,3-diaiole (hereinafter referred to as NBD) to give its derivative after the column and detecting the resulting derivative (post-column derivative method); a method of previously reacting the product in the reaction mixture with NBD to give its derivative therein followed by isolating and detecting the resulting derivative with NBD by means of reversed-phase chromatography with HPLC (pre-column derivative method) [see William J. Lindblad and Robert F. Diegelmann, Analytical Biochemistry, Vol. 138, pp. 390-395, 1984], etc. The detection of the derivative with NBD is conducted by measuring its luminescence (at the excited wavelength of 503 nm and the fluorescent wavelength of 541 nm) in both cases.

The enzyme may be isolated and purified in a usual manner from the culture of the transformant in which the formation of L-proline-3-hydroxylase is confirmed in the cultivated cell as mentioned above. For instance, the culture broth of the transformant is centrifuged to collect the cultivated cells therefrom, and the cells are washed and then disrupted by an ultrasonic cell disrupter, a French press, a Manton-Gauline homogenizer, a Dyno mill or the like to obtain a cell-free extract. The purified enzyme preparation can be obtained by ammonium sulfate precipitation, anion exchange chromatography such as diethylaminoethyl (DEAE) Sepharose or the like, hydrophobic chromatography such as butyl-Sepharose, phenyl-Sepharose or the like, gel filtration, electrophoresis such as isoelectric point electrophoresis, and so on from the supernatant of the cell-free extract obtained by centrifugation.

The cultivated transformant cells that have been identified to contain the L-proline-3-hydroxylase as formed therein can be cultivated under the same conditions as above, under which the transformant was cultivated, to thereby make the cells produce and accumulate cis-3-hydroxy-L-proline in the cells, and the thus-produced cis-3-hydroxy-L-proline can be collected from the culture to obtain it.

If the transformant cells derived from host cells which have the ability of producing L-proline from saccharide sources and accumulating it in the cultures and where such cells are used, it is possible to produce cis-3-hydroxy-L-proline even if L-proline is not added to the media during the cultivation of the cells therein. However, it is desirable to suitably add to the media L-proline at a concentration of from 5 to 1000 mM, preferably from 20 to 200 mM, whereby the intended L-proline-3-hydroxylases can be produced more efficiently.

If the transformant cells have the ability of producing 2-ketoglutaric acid from saccharide sources and accumulating it in the cultures and where such cells are used, it is possible to produce cis-3-hydroxy-L-proline even if 2-ketoglutaric acid is not added to the media during the cultivation of the cells therein. Where such transformant cells are used, saccharide sources such as glucose, etc. may be suitably added to the media to make the cells produce and accumulate 2-ketoglutaric acid in the cultures, whereby the intended L-proline-3-hydroxylases can be produced more efficiently. Where, on the other hand, transformant cells not having the ability of producing 2-ketoglutaric acid from saccharide sources are used, 2-ketoglutaric acid may be added to the media during the cultivation of the cells, if desired.

If desired, divalent iron ions may be added to the media during the cultivation of the transformant cells.

To produce cis-3-hydroxy-L-proline, also employable is another method to be mentioned below using, as the enzyme source, the cultures of the transformant cells where the formation of L-proline-3-hydroxylases has been identified, the cells isolated from the cultures, or the products as obtained by processing the cells.

The method to produce cis-3-hydroxy-L-proline is as follows: The cultures of the transformant cells, the cells isolated from the culture, or the products as obtained by processing the cells are added to aqueous media suitable for enzymatic reaction, along with L-proline, divalent iron ions and 2-ketoglutaric acid and optionally with surfactants and organic solvents, thereby converting L-proline into cis-3-hydroxy-L-proline, and thereafter the resulting cis-3-hydroxy-L-proline is collected from the reaction mixtures.

As examples of the processed cells, dried cells, lyophilized cells, surfactant-treated cells, enzymatically-treated cells, ultrasonically-treated cells, mechanically-ground cells, mechanically-compressed cells, solvent-treated cells, fractionated cell proteins, immobilized cells, immobilized materials obtained by processing their cells, etc. can be used. The enzyme preparations obtained by extraction from the cells having L-proline-3-hydroxylase activity, purified products of these enzyme preparations, and immobilized products thereof can also be used.

As examples of the aqueous medium, water, buffers such as phosphates, carbonates, acetates, borates, citrates and tris-buffers, alcohols such as methanol and ethanol, esters such as ethyl acetate, ketones such as acetone, and amides such as acetamide can be mentioned.

As examples of the surfactant, cationic surfactants such as polyoxyethylene-stearylamine (for example, Nymeen S215 produced by Nippon Oils & Fats Co.), cetyltrimethylammonium bromide, Cation FB, Cation F2-40E, etc.; anionic surfactants such as sodium oleylamidosulfate, Newrex TAB, and Rapizole 80.; ampholytic surfactants such as polyoxyethylene-sorbitan monostearate (for example, Nonion ST221) or the like; and also other tertiary amines PB, hexadecyldimethylamine, etc. can be mentioned. Any and every surfactant that promotes the reaction may be employed. The concentration of the surfactant is usually from 0.1 to 50 mg/liter, preferably from 1 to 20 mg/liter.

As examples of the organic solvent, toluene, xylene, aliphatic alcohols, benzene and ethyl acetate can be mentioned. The concentration of the organic solvent is usually from 0.1 to 50 µl/ml, preferably from 1 to 20 µl/ml.

The reaction may be conducted during the cultivation of the transformant having the activity of L-proline-3-hydroxylase, or may also be conducted after the completion of the cultivation, in the aqueous medium using the cells, the treated cells, the purified enzyme or the crude enzyme prepared from the culture.

The activity of the enzyme added to the reaction mixture is determined depending on the amount of the substrate used. It is usually from 1,000 to 10,000,000 U/liter, preferably from 10,000 to 3,000,000 U/liter. In case of using the cells or the treated cells of the microorganism, the concentration of wet cells is usually from 1 to 300 g/liter.

The reaction is usually conducted at a temperature from 15 to 50°C at a pH from 6.0 to 9.0 for 1 to 96 hours.

The concentration of L-proline used in the reaction may be from 1 mM to 2 M. L-Proline can be supplied by adding L-proline itself to the reaction mixture, or adding the culture of the microorganism which can produce and accumulate L-proline from sugar source. Further, if a microorganism having the ability of producing L-proline from a sugar source is used as the host microorganism of the transformant, L-proline produced from a sugar source by the host microorganism can be used in the reaction.

The divalent iron ion is required for the reaction. This divalent iron ion is ordinarily used in a concentration of from 1 to 100 mM. Any divalent iron ion can be used so long it does not inhibit the reaction. As examples of the divalent iron ion, sulfates such as ferrous sulfate; chlorides such as ferrous chloride; ferrous carbide; and organic acid salts such as citrates, lactates and fumarates can be mentioned. When the divalent iron ion is contained in the cells, the treated cells or the reaction mixture, the divalent iron ion need not be added.

2-Ketoglutaric acid itself may be added to the reaction mixture or may be supplied from a compound which can be converted into 2-ketaglutaric acid by the metabolic activity of the cells or the treated cells used. As examples of such a compound, saccharides such as glucose; amino acids such as glutamic acid; and organic acids such as succinic acid can be mentioned. These compounds may be used singly or in combination.

Cis-3-hydroxy-L-proline is recovered from the culture or the aqueous medium by any ordinary separation method, for example, column chromatography using an ion-exchange resin, crystallization, etc.

The structure of the thus-recovered cis-3-hydroxy-L-proline can be identified by ordinary analytical method such as ¹³C-NMR spectrum, ¹H-NMR spectrum, mass spectrum, specific rotation or the like.

The cis-3-hydroxy-L-proline produced by the present invention can be determined quantitatively by the above-mentioned post-column derivatization method or pre-column derivatization method.

### BRIEF DESCRIPTION OF THE DRAWINGS

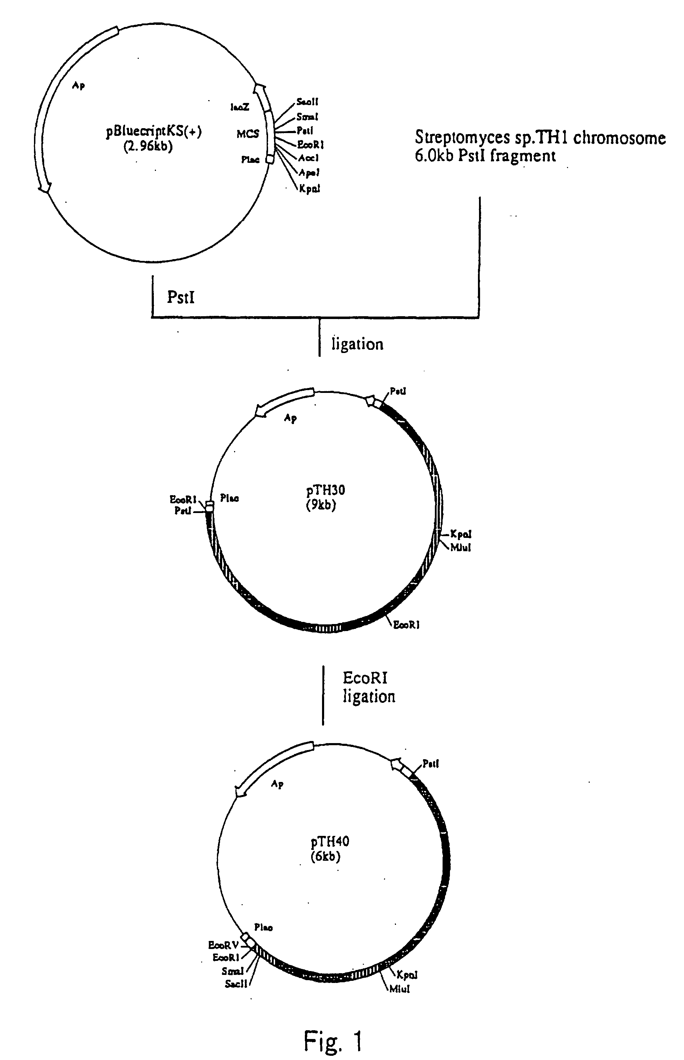

Fig. 1 shows the steps of constructing plasmid pTH30 and plasmid pTH40.

In this, the thick, solid black lines each indicate a cloned Streptomyces sp. TH1 chromosome site. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmids are shown.

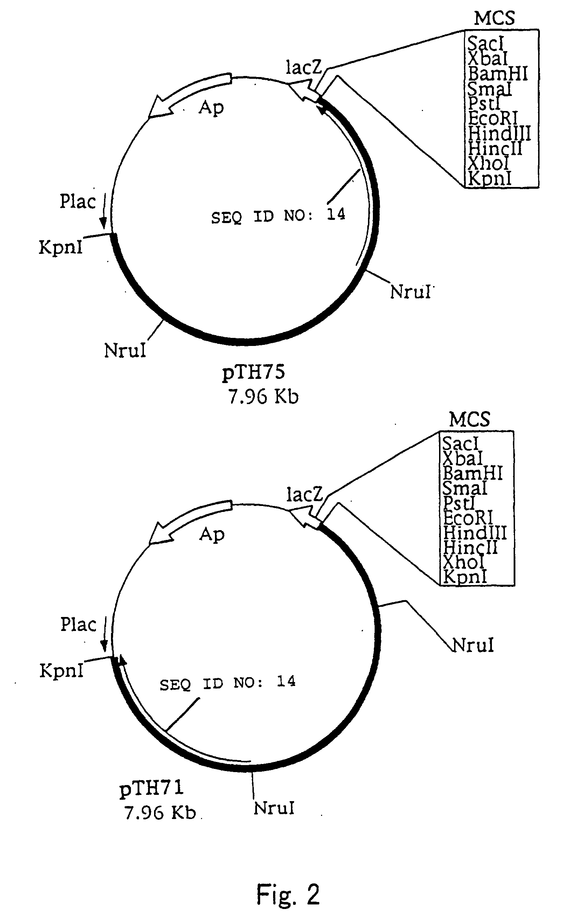

Fig. 2 shows plasmid pTH71 and plasmid pTH75.

In this, the thick, solid black lines each indicate a cloned Streptomyces sp. TH1 chromosome site. At the part of the thick, solid black line as drawn along with an arrow, the site has the base sequence corresponding to Sequence No. 14. The direction of each arrow indicates the direction toward the terminal of the sequence of Sequence No. 14 from the head thereof. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmids are shown.

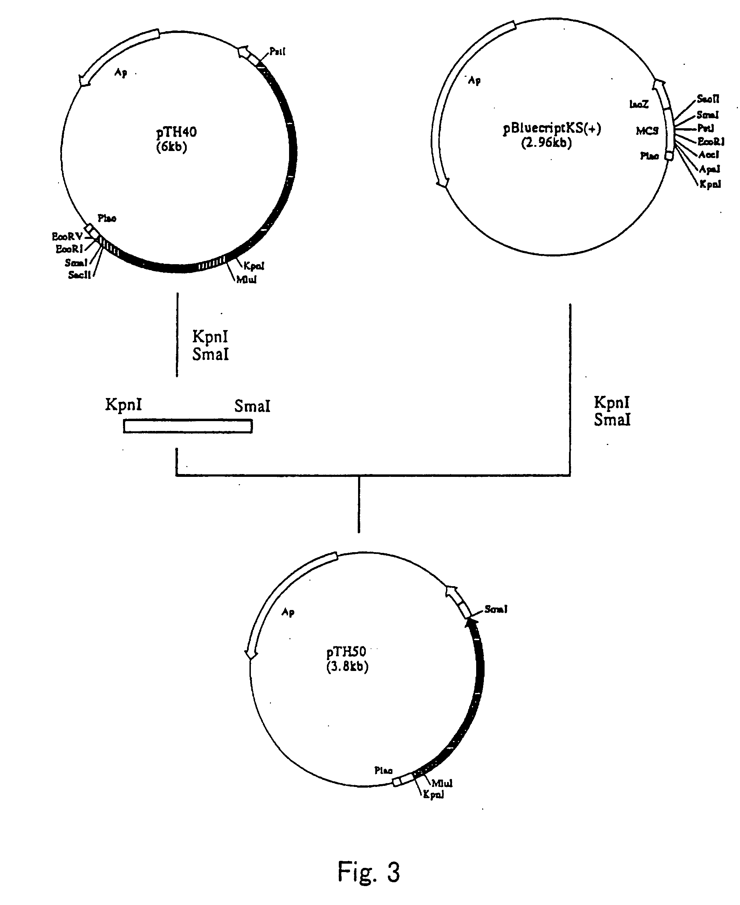

Fig. 3 shows the steps of constructing plasmid pTH50.

In this, the thick, solid black lines each indicate a part containing a Streptomyces sp. TH1 chromosome site-derived L-proline-3-hydroxylase gene. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmid are shown.

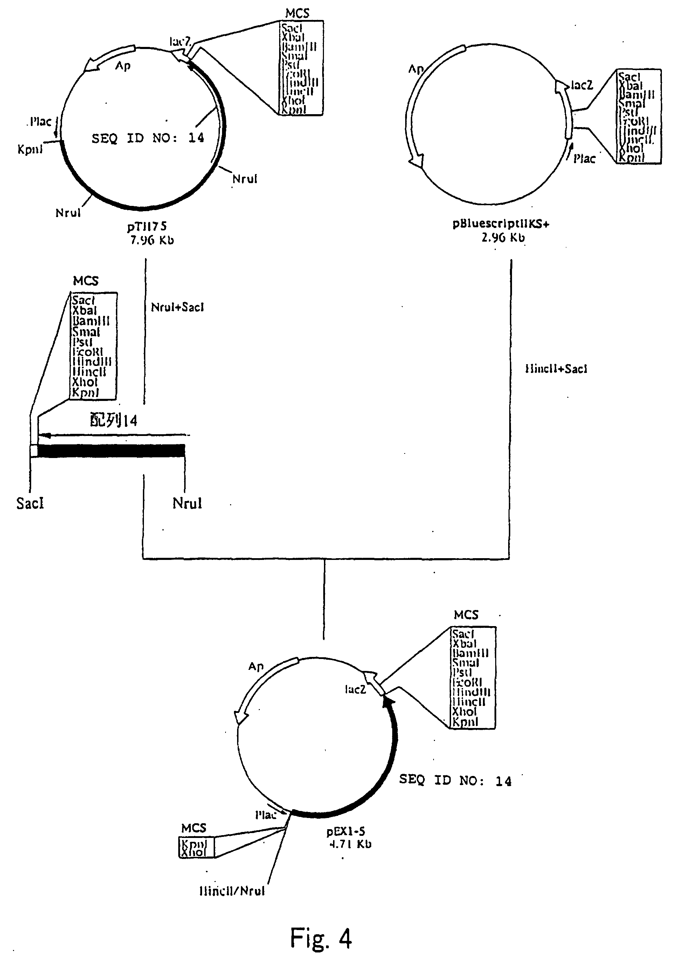

Fig. 4 shows the steps of constructing plasmid pEX1-5.

In this, the thick, solid black lines each indicate a part containing a Streptomyces sp. TH1 chromosome site-derived L-proline-3-hydroxylase gene. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmid are shown.

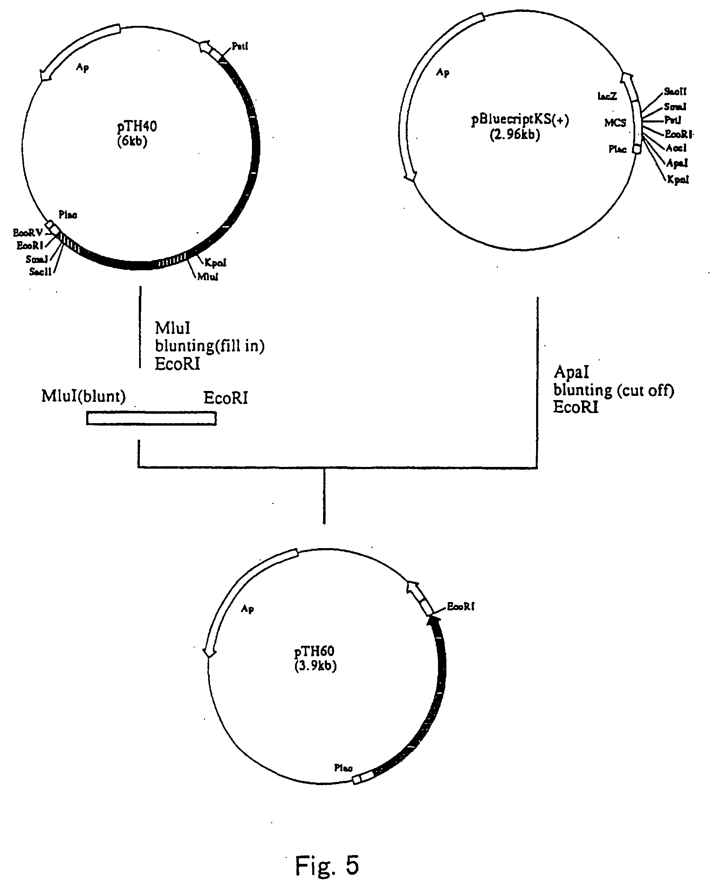

Fig. 5 shows the steps of constructing plasmid pTH60.

In this, the thick, solid black lines each indicate a part containing a Streptomyces sp. TH1 chromosome site-derived L-proline-3-hydroxylase gene. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmid are shown.

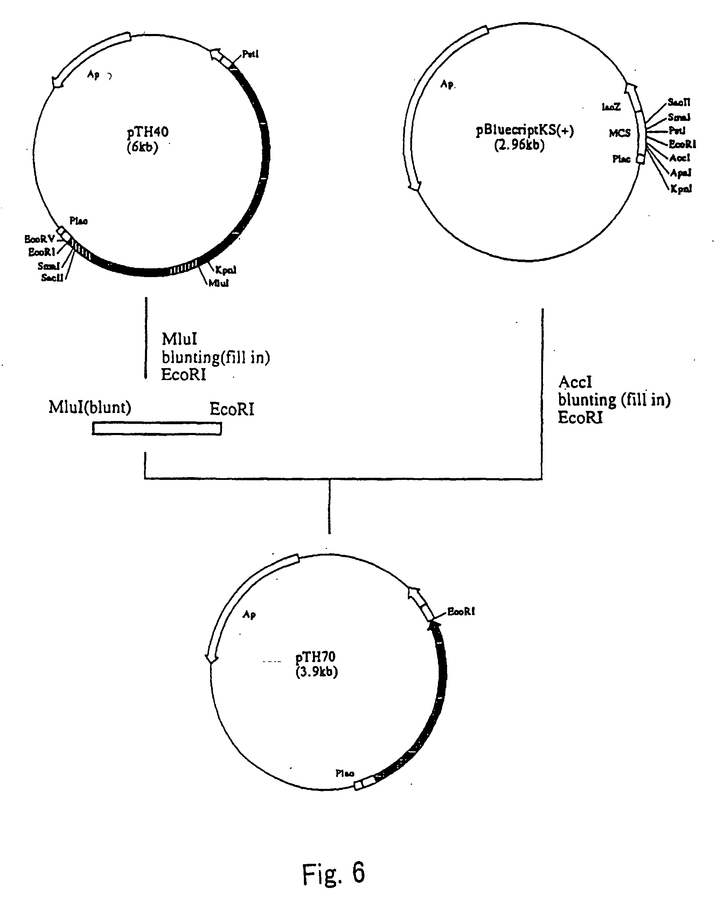

Fig. 6 shows the steps of constructing plasmid pTH70.

In this, the thick, solid black lines each indicate a part containing a Streptomyces sp. TH1 chromosome site-derived L-proline-3-hydroxylase gene. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmid are shown.

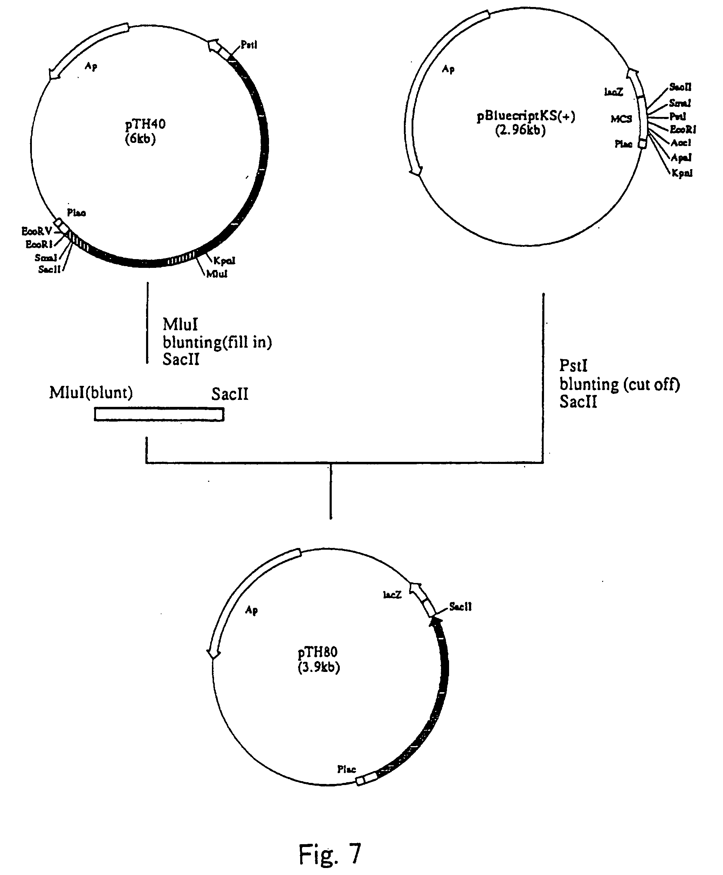

Fig. 7 shows the steps of constructing plasmid pTH80.

In this, the thick, solid black lines each indicate apart containing a Streptomyces sp. TH1 chromosome site-derived L-proline-3-hydroxylase gene. Ap indicates a pBR322-derived ampicillin-resistant gene. Plac indicates an Escherichia coli lactose promoter; lacZ indicates a β-galactosidase structural gene; and MCS indicates a multi-cloning site. In this, only the restriction enzyme sites having relation to the construction of the plasmid are shown.

### BEST MODES OF CARRYING OUT THE INVENTION

### Example 1: Preparation of partial DNA fragment of the gene encoding L-proline -3-hydroxylase protein derived from Streptomyces sp. TH1:

### (1) Isolation of Chromosomal DNA of Streptomyces sp. TH1:

Chromosomal DNA of Streptomyces sp. TH1 was isolated in the usual manner as follows. SK#2 medium (comprising 0.25 % glucose, 1.0 % soluble starch, 0.25 % yeast extract, 0.25 % peptone, 0.15 % meat extract, 0.01 % potassium dihydrogen phosphate and 0.03 % magnesium sulfate, and adjusted to a pH 7.6 with 6 N NaOH) containing 5 % mannitol and 0.05 % glycine, was put in test tubes in an amount of 10 ml each, and sterilized at 120°C for 20 minutes. One loopful of cells of Streptomyces sp. TH1 which had grown in HT-agar plate medium comprising 1% soluble starch, 0.2% NZ amine, 0.1% yeast extract, 0.1% meat extract and 1.5 % agar, and adjusted to pH 7.2 with 6N NaOH was inoculated in the above-mentioned SK#2 medium, and cultivated at 28°C for 3 days with shaking.

The culture was centrifuged, and the obtained cells were washed with 10 ml of a 10.3 % sucrose solution, and suspended in 6 ml of TS comprising 10.3 % sucrose, 50 mM tris-HCl (pH 8.0) and 25 mM EDTA. One milliliter of a lysozyme solution (50 mg/ml·TS) was added thereto, and the mixture was incubated at 37°C for 60 minutes.

Subsequently, 0.6 ml of a Proteinase K (produced by Sigma Co.) solution (2 mg/ml·TS) was added to the lysozyme-treated solution, and gently stirred. Further, 3.6 ml of a 3.3% (w/v) SDS solution was added thereto while gently mixing, and the mixture was incubated at 37°C for 60 minutes. The mixture was heated at 50°C for 30 minutes, and then cooled with water. An equal amount of TE [containing 10 mM tris-HCl (pH 8.0) and 1 mM EDTA] saturated phenol-chloroform (1/1, v/v) was added thereto, and the mixed solution was moderately shaked for 30 minutes. After the centrifugation, the upper layer was taken, and again subjected to extraction with the mixture of TE saturated phenol-chloroform. The extract was centrifuged, and an equal amount of chloroform was then added to the upper layer, and mixed. The mixture was recentrifuged.

The upper layer was taken, and 20 µl of an RNase A aqueous solution (10 mg/ml) heat-treated at 100°C for 10 minutes were added to the upper layer. The mixture was incubated at 37°C for 45 minutes. To the RNase A-treated solution were added 1/10 volume of a 5 M NaCl aqueous solution and 1/4 volume of 50 % PEG6000, and gently mixed. The mixture was allowed to stand overnight while being cooled with ice. After the mixed solution was centrifuged at 12,000 rpm for 10 minutes, the supernatant was discarded completely, and the precipitate was dissolved in 5 ml of TE. After 1/10 volume of a 3 M sodium acetate solution and 1/30 volume of a 66 mM magnesium chloride solution were added thereto and mixed, 2.2 volumes of cold ethanol were added, and gently mixed. After the mixed solution was centrifuged at 10,000 rpm for 10 minutes, the supernatant was discarded, and the precipitate was washed twice with 70 % cold ethanol. The precipitate containing 250 µg of chromosomal DNA was dissolved in TE and used in the subsequent experiment as chromosomal DNA.

### (2) Determination of Partial Amino Acid Sequence of Streptomyces sp. TH1-derived L-proline-3-hydroxylase Protein:

L-proline-3-hydroxylase produced by Streptomyces sp. TH1 was isolated and purified according to the method described in Reference Example 1, and the N-terminal amino acid sequence of the protein of the purified enzyme was analyzed, using Protein Sequencer Model PPSQ-10 (produced by Shimadzu Seisakusho Co.), to determine the N-terminal amino acid sequence thereof as indicated by Sequence No. 5.

Furthermore, the purifed enzyme obtained in Example 5 was treated with lysyl endopeptidase in 0.1 M Tris-HCl with 4 M urea (pH 9), at 37°C for 6 hours, and the thus-obtained peptide fragments were collected through HPLC. Their amino acid sequences were also analyzed in the same manner as above to determine the partial amino acid sequences as indicated by Sequence Nos. 6 and 7.

### (3) Preparation of Partial DNA of L-proline-3-hydroxylase Gene:

A sense chain mix DNA primer as indicated by Sequence No. 8, which corresponds to the sequence of from the 1st to 6th amino acids in the amino acid sequence as indicated by Sequence No. 5, and an anti-sense chain mix DNA primer as indicated by Sequence No. 9, which corresponds to the sequence of from the 24th to 28th amino acids in the amino acid sequence as indicated by Sequence No. 5, were synthesized, using 380A DNA Synthesizer (produced by Applied Biosystems Co.).

Using the above-synthesized DNAs as primers and the Streptomyces sp. TH1 chromosome DNA as template, the PCR was conducted using DNA Thermal Cycler 480 (produced by Perkin Elmer Cetus Co.).

The reaction was conducted using 20 µl of a reaction mixture having the following formulation.

Formulation of the reaction mixture:: 21 ng/µl Streptomyces sp. TH1 chromosome DNA, 10 µM sense chain mix DNA primer, 10 µM anti-sense chain mix DNA primer, 0.125 U/µl Pfu DNA polymerase (produced by STRATAGENE Co.), 10 % DMSO, 20 mM Tris-HCl (pH 8.2), 10 mM KCl, 6 mM ammonium sulfate, 2 mM magnesium chloride, 0.1 % Triton X-100, 10 ng/µl bovine serum albumin.

After the completion of an incubation at 95°C for 5 minutes, a three step incubation, namely at 95°C for 0.5 minute, at 25°C for 0.5 minute and at 75°C for 1 minute was repeated for a total of 40 times. The reaction mixture was subjected to electrophoresis with 15% polyacrylamide gel (PAGEL NPU-15L produced by Atto Co.), and the amplified 83 bp DNA was recovered using da Vinci Kun (Pen Touch Recovery NB-7000 Model) manufactured by Nippon Eido K.K.

Using the recovered DNA fragment (83 bp) as a template, the sense strand mix DNA primer indicated in Sequence No. 8 as a primer and the anti-sense strand mix DNA primer indicated in Sequence No. 10 corresponding to amino acids Nos. 21st to 25th of an amino acid sequence indicated in Sequence No. 5 as a primer, the PCR was conducted again in the same manner, and the amplified 74 bp DNA was recovered. The thus-recovered DNA fragment was inserted into a Sma I site of pUC18 using Sure Clone Ligation Kit (produced by Pharmacia Co.), and the nucleotide sequence was determined by a nucleotide sequencing kit (Taq DyeDeoxy™ Terminator Cycle Sequencing Kit produced by Applied Biosystems).

The nucleotide sequences thus determined are indicated by Sequence Nos. 11 and 12. Thus, the two nucleotide sequences were determined. The amino acid sequence to be presumed from the nucleotide sequence of Sequence No. 11 completely corresponded to the N-terminal amino acid sequence of the pure enzyme as indicated by Sequence No. 5, except for the 2nd amino acid that could not be identified in the sequencing of the N-terminal amino acid sequence. The amino acid sequence to be presumed from the nucleotide sequence of Sequence No. 12 exhibited high homology to the N-terminal amino acid sequence of the pure enzyme as indicated by Sequence No. 5.

### Example 2: Cloning of a DNA fragment containing L-proline-3-hydroxylase gene:

### (1) Preparation of a digoxigeniated (DIGated) probe:

The two 74 bp fragments obtained in (3) of Example 1 and indicated by Sequence Nos. 11 and 12 each were inserted into the Sma I site of pUC18. Using each of these two plasmids thus obtained as a template, each plasmid was subjected to PCR in the same manner as in (3) of Example 1, using 50 µl of a reaction mixture containing 10 µM of the sense chain synthetic DNA as indicated by Sequence No. 8 and 10 µM of the anti-sense chain synthetic DNA as indicated by Sequence No. 10. The resulting reaction mixture each were subjected to 12.5 % polyacrylamide gel electrophoresis to identify the formation of an amplified 74 bp fragment. From each gel, the amplified fragment was recovered in the same manner as in (3) of Example 1.

The 74 bp fragment was digoxigeniated (DIGated), using PCR DIG Labeling Kit (produced by Boehringer Mannheim Co.).

Using each of the thus-recovered two 74 bp fragments as a template, each fragment was subjected to PCR, using 2.5 U of Pfu DNA polymerase (produced by STRATAGENE Co.), 5 µl of x10 buffer for Pfu DNA polymerase (produced by STRATAGENE Co.), 5 µl of DMSO, 5 µl of x 10 PCR DIG mix (produced by Boehringer Mannheim Co.) and 50 µl of a reaction mixture comprising 10 µM of the sense chain synthetic DNA of Sequence No. 8 and 10 µM of the anti-sense chain synthetic DNA of Sequence No. 10.

After the incubation at 95°C for 5 minutes, the step of the incubation at 95°C for 0.5 minute, at 50°C for 0.5 minute and at 75°C for 1 minute was repeated 40 times. After the reaction, each reaction mixture was subjected to 12.5 % polyacrylamide gel electrophoresis to identify the formation of an amplified 74 bp fragment. From each gel, was recovered the amplified fragment in the same manner as in (3) of Example 1. The resulting two amplified fragments were used as probes. The probe derived from the nucleotide sequence of Sequence No. 11 is referred to as probe A, and that from the nucleotide sequence of Sequence No. 12 as probe B.

### (2) Southern Hybridization:

Twenty units of a restriction enzyme Pst I (produced by Takara Shuzo Co., LTD) was added to 20 µg of Streptomyces sp. TH1 chromosome DNA and reacted at 37°C for 2 hours to cleave the DNA, which was then subjected to agarose gel electrophoresis. Using the probe A as obtained in (1) of Example 2, Southern hybridization was conducted with DIG Luminescent Detection Kit (produced by Boehringer Mannheim Co.) according to the method as described in the description attached to the kit.

Precisely, after the completion of the agarose gel electrophoresis, the gel was gently shaken in 0.25 N HCl for 20 minutes and then blotted onto Hybond-N⁺ membrane (produced by Amersham Co.) in 0.4 M sodium hydroxide solution, while being sucked at 7.5 mmHg using Genopirator Pump AE-6680P (produced by ATTO Co.) and Genopirator AE-6680C (produced by ATTO Co.). The thus-blotted membrane was dried in air. The resulting membrane was dipped in 10 ml of a hybridization buffer of DIG Luminescent Detection Kit [comprising 50 % v/v formamide, 2 % a blocking reagent, 0.1 % w/v N-laurylsarcosine and 0.02 % w/v SDS in x5 SSC (x1 SSC is comprised of 150 mM sodium chloride and 15 mM sodium citrate)] at 42°C for 1 hour and thereafter dipped in a probe solution [prepared by adding 3 µl of the probe A as obtained in (1) of Example 2 to 200 µl of the hybridization buffer, then heating the mixture at 95°C for 2 hours and thereafter adding thereto the hybridization buffer until the total volume was 1.5 ml], at 42°C overnight. The thus-obtained membrane was washed twice each with 25 ml of x2 SSC containing 0.1 % SDS for 5 minutes at room temperature and then twice each with 25 ml of x0.1 SSC containing 0.1 % SDS for 15 minutes at 68°C.

After this, the membrane was treated with a washing buffer [buffer 1 (0.1 M maleic acid, 0.15 M sodium chloride, pH 7.5) containing 0.3 % w/v Tween-20] for 1 to 5 minutes at room temperature, then with 50 ml of buffer 2 (buffer 1 containing 1 % blocking reagent) for 30 minutes at room temperature, then with 10 ml of buffer 2 containing 1 µl of anti-digoxigenin-AP Fab for 30 minutes at room temperature, then twice each with 50 ml of buffer 2 for 30 minutes at room temperature, then with 10 ml of buffer 3 (0.1 M Tris-HCl, 0.1 M sodium chloride, 50 mM magnesium chloride, pH 9.5) for from 2 to 5 minutes at room temperature, and then with 5 ml of buffer 3 containing 50 µl of Lumigen PPD for 5 minutes at room temperature, in that order. Subsequently, water was removed from the film quickly over a filter paper, then the film was wrapped with a wrapping film, Saran Wrap (poly vinylidene chloride film) and thereafter allowed to stand at 37°C for 15 minutes. Using Hyperfilm ECL (produced by Amersham Co.), the resulting film was exposed at room temperature for 30 minutes.

A DNA fragment as strongly hybridized with the probe was detected at the position of approximately 6 kb.

The same process as in (2) of Example 2 was repeated, while using the probe B as obtained in (1) of Example 2 and while using 20 U of a restriction enzyme KpnI (produced by Takara Shuzo Co., LTD) in place of the restriction enzyme PstI. As a result, a DNA fragment as strongly hybridized with the probe was detected at the position of approximately 5 kb.

### (3) Fractionation of Chromosome DNA:

Twenty units of a restriction enzyme PstI (produced by Takara Shuzo Co., LTD) was added to 20 µg of Streptomyces sp. TH1 chromosome DNA and the mixture was allowed to stand at 37°C for 2 hours to cleave the DNA, which was then mixed with the same amount, as that of the reaction mixture, of TE-saturated phenol/chloroform. The resulting mixture was centrifuged, and the upper layer was taken out. To the layer was added 2.2 times volume of cold ethanol and then gently mixed. This mixture was centrifuged at 10,000 rpm for 10 minutes, the supernatant was removed, and the precipitate was washed twice with 70 % cold ethanol to obtain an ethanol precipitate. The process of obtaining the ethanol precipitate by using TE-saturated phenol/chloroform and cold ethanol is hereinafter referred to as an ethanol precipitation. The ethanol precipitate was dissolved in 120 µl of TE and subjected to agarose gel electrophoresis. After the completion of the electrophoresis, a DNA fraction at approximately 6 kb was extracted from the agarose gel and purified by the use of Prep-A-Gene (produced by Biorad Co.). Thus approximately 0.5 µg of a PstI-cleaved chromosome DNA fraction was obtained.

The same process as in (3) of Example 2 was repeated, except using 20 U of a restriction enzyme KpnI in place of PstI. Thus was obtained approximately 0.5 µg of a KpnI-cleaved chromosome DNA fraction at approximately 5 kb.

### (4) Formation of Plasmid Library:

1) After 1 µg of pBluescriptII KS(+) (produced by STRATAGENE Co.) was cleaved with restriction enzyme PstI (produced by Takara Shuzo Co., LTD) and then dephosphorylated with an alkaline phosphatase (derived from calf intestines) (produced by Takara Shuzo Co.,LTD) in accordance with the method described in the description attached to the product. After the completion of the reaction, the reaction mixture was subjected to the ethanol precipitation to obtain an ethanol precipitate. The precipitate was dissolved in 5 µl of TE. The thus-obtained, PstI-cleaved pBluescriptII KS(+) DNA was reacted with 0.1 µg of PstI-cleaved chromosome DNA as obtained in (3) of Example 2, using a ligation kit (TAKARA Ligation Kit, produced by Takara Shuzo Co., LTD), for 2.5 hours at 26°C, whereby the two DNAs were ligated to each other. Using the thus-ligated DNA, E. coli XL2-Blue was transformed in a usual manner. The resulting transformant were spread on LB-agar medium containing 50 µg/ml ampicillin and cultivated thereon overnight at 37°C to obtain about 240 colonies.
2) After 1 µg of pBluescriptII KS(+) (produced by STRATAGENE Co.) was cleaved with a restriction enzyme KpnI (produced by Takara Shuzo Co., LTD), dephosphorylation was conducted with an alkaline phosphatase (derived from calf intestines) (produced by Takara Shuzo Co., LTD) in accordance with the method described in the description attached to the product. After the completion of the reaction, the reaction mixture was subjected to the ethanol precipitation to obtain an ethanol precipitate. The precipitate was dissolved in 5 µl of TE. The thus-obtained, KpnI-cleaved pBluescriptII KS(+) DNA was reacted with 0.1 µg of KpnI-cleaved chromosome DNA as obtained in (3) of Example 2, using a ligation kit (TAKARA Ligation Kit, produced by Takara Shuzo Co., LTD), for 2.5 hours at 26°C, whereby the two DNAs were ligated to each other. Using the thus-ligated DNA, E. coli XL2-Blue was transformed in a usual manner. The resulting transformants were spread on LB-agar medium containing 50 µg/ml of ampicillin and cultivated thereon overnight at 37°C to obtain about 200 colonies.

### (5) Selection of Intended Clone:

The colonies having the desired clone was selected from the above-obtained colonies as follows.

Precisely, the colonies appearing on the LB-agar medium were transferred onto a nylon membrane (Nytran, produced by Schleicher & Schuell Co.) that has been previously washed with x5 SSC, and the membrane was put on filter paper immersed with 0.5 M NaOH for 5 minutes. Subsequently, the film was put on filter paper immersed with 1.0 M Tris-HCl (pH 8.0) for 5 minutes and then on filter paper immersed with 1.5 M sodium chloride-1.0 M Tris-HCl (pH 8.0) for 5 minutes. After the film was lightly washed with x2 SSC, then crosslinking was conducted by exposure to ultraviolet rays (120 mJ/cm²). Then the surface of the film was wiped with x2 SSC containing 0.1 % of SDS, and the membrane was dried in air.

The detection was conducted using the DIGated probes as obtained in (1) of Example 2, and positive colonies were detected on the membrane DIG Luminescent Detection Kit (produced by Boehringer Mannheim Co.), in accordance with the process as described in (2) of Example 2.

Precisely, by the colony hybridization between about 240 colonies as obtained in 1) of Example 2(4) and the probe A as obtained in (1) of Example 2, one positive colony having the intended clone was detected. By the colony hybridization between about 200 colonies as obtained in 2) of Example 2(4) and the probe B as obtained in (1) of Example 2, five positive colonies each having the intended clone were detected.

From these colonies, plasmids were extracted in a usual manner. Thus were obtained pTH30, pTH71 and pTH75.

The structures of the plasmids were identified through digestion with restriction enzymes. The structure of pTH30 was such that a PstI-cleaved DNA fragment of approximately 6 kb was inserted into the PstI site of pBluescriptII KS(+) (see Fig. 1); while the structures of pTH71 and pTH75 were such that a KpnI-cleaved DNA fragment of approximately 5 kb was inserted into the KpnI site of pBluescriptII KS(+) in the opposite directions (see Fig. 2).

### (6) Determination of nucleotide Sequence:

1) Using a deletion kit for kilosequences (produced by Takara Shuzo Co., LTD), plasmid pTH30 was processed to give various deletion mutant plasmids. The reagents and the process as indicated in the description attached to the kit were employed. The nucleotide sequences of the deletion plasmids were analyzed, using a nucleotide sequencing kit (Taq DyeDeoxy™ Terminator Cycle Sequencing Kit, produced by Applied Biosystems Co.), and the nucleotide sequence of 1081 b KpnI-EcoRI fragment as indicated by Sequence No. 13 was identified.
   In the nucleotide sequence thus identified, the nucleotide sequence indicated by Sequence No. 3 that codes for the protein composed of 290 amino acid residues indicated by Sequence No. 1 was present. The amino acid sequence comprises the N-terminal amino acid sequence indicated by Sequence No. 5, which was determined by using the sequence of the pure L-proline-3-hydroxylase, and the internal amino acid sequences indicated by Sequence Nos. 6 and 7, which revealed the existence of the intended L-proline-3-hydroxylase gene in the PstI fragment of approximately 6 kb obtained hereinabove. The gene is hereinafter referred to as L-proline-3-hydroxylase I-type gene.
2) The nucleotide sequence of the Streptomyces-derived insertion fragment terminal in each of plasmids pTH71 and pTH75 was analyzed, using a base sequencing kit (Taq DyeDeoxy™ Terminator Cycle Sequencing Kit, produced by Applied Biosystems Co.). On the basis of the thus-sequenced nucleotide sequence, a sequencing primer was synthesized using 380A Model DNA Synthesizer (produced by Applied Biosystems Co.). Using the primer, the nucleotide sequence of a part of each of pTH71 and pTH75 existing downstream from the Streptomyces-derived insertion fragment was analyzed. On the basis of the thus-sequenced nucleotide sequence, another sequencing primer was synthesized. Using the primer, a part of each of the plasmids existing further downstream was analyzed. The base sequencing operation comprising the above-mentioned steps was repeated, by which the NruI-KpnI fragment of 1826 bp was sequenced as in Sequence No. 14.

The nucleotide sequence of the fragment thus sequenced comprised the nucleotide sequence indicated by Sequence No. 4 that codes for the protein composed of 290 amino acid residues as indicated by Sequence No. 2. The nucleotide sequence of Sequence No. 4 comprised the nucleotide sequence indicated by Sequence No. 12. The nucleotide sequence indicated by Sequence No. 4 was homologous at 78.5 % to the L-proline-3-hydroxylase I-type gene. The gene indicated by Sequence No. 4 is hereinafter referred to as L-proline-3-hydroxylase II-type gene.

### Example 3: Construction of L-proline-3-hydroxylase Expression Plasmid:

### (1) Construction of L-proline-3-hydroxylase I-type Gene Expression Plasmid:

Plasmid pTH30 as obtained in Example 2 was cleaved with a restriction enzyme EcoRI, and then self-ligated using a ligation kit (produced by Takara Shuzo Co., LTD). E. coli XL1-Blue MRF' strain was transformed with the DNA resulting from the self-ligation in a usual manner. A plasmid was extracted from the transformant in a usual manner, and the structure of the plasmid was identified through digestion with restriction enzyme.

As a result of the above, obtained was plasmid pTH40, from in which the EcoRI fragment of approximately 3 kb existing in the 3'-side of L-proline-3-hydroxylase gene was removed (see Fig. 1).

Subsequently, plasmid pTH40 was cleaved with KpnI and SmaI, then a fragment of approximately 0.95 kbp containing L-proline-3-hydroxylase I-type gene was identified through agarose gel electrophoresis, and the fragment was recovered from the agarose gel in a usual manner. The KpnI-SmaI-cleaved fragment was inserted into the KpnI-SmaI-cleaved site of pBluescriptII KS(+), using a ligation kit (produced by Takara Shuzo Co., LTD). With the resulting DNA, E. coli XL1-Blue MRF' strain was transformed in a usual manner. The resulting transformant was spread on LB-agar medium containing 50 µg/ml ampicillin and cultivated thereon overnight at 37°C. A plasmid was extracted from the colonies of the transformant thus grown in a usual manner, and its structure was identified through digestion with restriction enzyme.

As a result of the above, obtained was plasmid pTH50, into which the DNA fragment coding for L-proline-3-hydroxylase I-type gene was inserted in the same direction as that for transcription of lac promoter (see Fig. 3).

### (2) Construction of L-proline-3-hydroxylase II-type Gene Expression Plasmid:

Plasmid pTH75 as obtained in Example 2 was cleaved with restriction enzymes NruI and SacI, and thus-cleaved fragment of approximately 1900 bp containing L-proline-3-hydroxylase II-type gene was recovered through agarose gel electrophoresis. The NruI-SacI-cleaved fragment was inserted into the HincII-SacI-cleaved site of pBluescriptII KS(+), using a ligation kit (produced by Takara Shuzo Co., LTD). With the resulting DNA, E. coli XL2-Blue MRF' strain was transformed in a usual manner. The resulting transformant was spread on LB-agar medium containing 50 µg/ml ampicillin and cultivated thereon overnight at 37°C. A plasmid was extracted from the colonies of the transformant thus grown in a usual manner, and its structure was identified through digestion with restriction enzyme.

As a result of the above, obtained was plasmid pEX1-5, into which the DNA fragment coding for L-proline-3-hydroxylase II-type gene was inserted in the direction same as that for transcription of lac promoter (see Fig. 4).

### Example 4: Production of L-proline-3-hydroxylase by Transformants:

### (1) Production of L-proline-3-hydroxylase by Transformant Having L-proline-3-hydroxylase I-type Gene:

E. coli ATCC12435 was transformed with plasmid pTH50 as obtained in Example 3. The resulting transformant was inoculated in 3 ml of an LB medium containing 50 µg/ml ampicillin and cultivated therein overnight at 30°C by shaking. The resulting culture was centrifuged and the thus-obtained wet cells were frozen at -20°C.

After the fozen cells were thawed, thus-obtained cells were added to 250 µl of a reaction mixture [containing 12 mM L-proline, 24 mM 2-ketoglutaric acid, 4 mM ferrous sulfate and 8 mM L-ascorbic acid in 200 mM TES buffer (pH 7.5)] at 4 % (w/v) in terms of the wet cells, and the mixture was allowed to stand for 10 minutes at 35°C. The reaction mixture was heated at 100°C for 2 minutes to stop the reaction.

The resulting reaction mixture was centrifuged, and 100 µl of 0.3 M borate buffer (pH 10.7), 4 µl of 10 % (v/v) mercaptoethanol aqueous solution and 16 µl of 5 % (w/v) OPA in ethanol were added to 100 µl of the resulting supernatant and left at 60°C for 30 seconds, and then 50 µl of 2 % (w/v) NBD in ethanol was added thereto. The mixture was allowed to stand at 60°C for 40 minutes. Then 30 µl of 1 N hydrochloric acid was added to the reaction mixture to stop the reaction. The resulting reaction mixture was centrifuged and filtered through a filter to remove the precipitate therefrom, and the resulting filtrate was analyzed through HPLC by which the product cis-3-hydroxy-L-proline produced was quantitatively determined.

HPLC was conducted under the following conditions.
Mobile Phase: 10 mM citric acid (pH 4.0)/methanol = 3/1 (v/v).
Flow Rate: 1 ml/min.
Column: YMC Pack ODS AQ-312
   (produced by YMC Co., 6 x 150 mm).
Column Temperature: 50°C.
Detection: fluorophotometry
   excitation wavelength : 503 nm
   emission wavelength : 541 nm.

As is shown in Table 1 below, the transformant E. coli ATCC12435/pTH50 produced L-proline-3-hydroxylase more by approximately 34 times/cell than the Streptomyces sp. TH1 strain which had been used as the gene source.

**Table 1**

| L-proline-3-hydroxylase Activity Produced by Transformant | | |
|---|---|---|
| Strain | Cell Activity¹⁾ | Relative Activity²⁾ |
| E. coli ATCC12435/pTH50 | 1.94 | 34.0 |
| E. coli ATCC12435/pBluescriptII KS(+) | Not detected. | - |
| Streptomyces sp. TH1³⁾ | 0.057 | 1 |

| | | |
|---|---|---|
| 1) Cell activity indicates the enzymatic activity per mg of wet cells (U/mg wet cells). One U indicates the enzymatic activity of producing 1 nmol of cis-3-hydroxy-L-proline per minute (nmol/min). | | |
| 2) Relative activity is based on the enzymatic activity produced by Streptomyces sp. TH1 strain of being 1 (one). | | |
| 3) This is referred to in Reference Example 2. | | |

### (2) Production of L-proline-3-hydroxylase by Transformant Having L-proline-3-hydroxylase II-type Gene:

E. coli ATCC12435 was transformed with plasmid pEX1-5 as obtained in Example 3. The resulting transformant was inoculated in 3 ml of a TB medium containing 50 µg/ml ampicillin and cultivated therein overnight at 30°C by shaking. The resulting culture was centrifuged, and the cells were collected. If desired, the cells were frozen and stored at -20°C, and the frozen cells were thawed before use.

The cells were added to 500 µl of a reaction mixture [containing 25 mM L-proline, 50 mM 2-ketoglutaric acid, 4 mM ferrous sulfate and 8 mM L-ascorbic acid in 200 mM TES buffer (pH 7.5)] at 2.2 % (w/v) in terms of the wet cells, and reacted for 15 minutes at 35°C. The reaction mixture was heated at 100°C for 2 minutes to stop the reaction.

The resulting reaction mixture was centrifuged, and 100 µl of 0.3 M borate buffer (pH 10.7), 4 µl of 10 % (v/v) mercaptoethanol aqueous solution and 16 µl of 5 % (w/v) OPA in ethanol were added to 100 µl of the resulting supernatant and left at 60°C for 30 seconds, and thereafter the mixture was neutralized. This treatment resulted in modification of primary amino acids with OPA but not the secondary amino acid, hydroxyproline. The thus-processed reaction mixture was analyzed through HPLC, by which the product of cis-3-hydroxy-L-proline produced was quantitatively determined. HPLC was conducted under the following conditions.
Mobile Phase: aqueous solution of 1 mM copper sulfate.
Flow Rate: 1 ml/min.
Column: SUMICHIRAL OA-5000 (produced by Sumika Chemical Analysis Service Co., 4.6 x 250 mm)
Column Temperature: 38°C.

The eluate as optically resolved under the conditions mentioned above was on-line mixed with reactants mentioned below under the conditions mentioned below, in which the eluate was modified with NBD in a reaction box CRB-6A (produced by Shimadzu Co.) and the NBDated-secondary amino acid was quantitatively determined through fluorophotometry.

Reactants and Amounts Thereof Added:
aqueous solution of 300 mM boric acid and
   25 mM EDTA (pH 9.6) 0.2 ml/min.
1 g/liter methanol of NBD: 0.5 ml/min.
Reaction Temperature: 60°C.
Detection: fluorophotometry
   excitation wavelength 503 nm
   emission wavelength 541 nm

As is shown in Table 2 below, the transformant E. coli ATCC12435/pEX1-5 produced L-proline-3-hydroxylase more by approximately 77 times/cell than the Streptomyces sp. TH1 strain which had been used as the gene source.

**Table 2**

| L-Proline-3-hydroxylase Activity Produced by Transformant | | |
|---|---|---|
| Strain | Cell Activity¹⁾ | Relative Activity²⁾ |
| E. coli ATCC12435/pEX1-5 | 4.42 | 77.5 |
| E. coli ATCC12435/pBluescriptII KS (+) | Not detected. | - |
| Streptomyces sp. TH1³⁾ | 0.057 | 1 |

| | | |
|---|---|---|
| 1) Cell activity indicates the enzymatic activity per mg of wet cells (U/mg wet cells). One U indicates the enzymatic activity of producing 1 nmol of cis-3-hydroxy-L-proline per minute (nmol/min). | | |
| 2) Relative activity is based on the enzymatic activity produced by Streptomyces sp. TH1 strain of being 1 (one). | | |
| 3) This is referred to in Reference Example 2. | | |

### Example 5: Construction of Expression Plasmid for a fused protein with a β-galactosidase protein fragment:

### (1) Construction of pTH60 Plasmid:

Four micrograms of pTH40 DNA was cleaved with MluI and subjected to ethanol precipitation to obtain an ethanol precipitate. The thus-obtained ethanol precipitate (DNA fragment) was dissolved in 36 µl of TE, and the both terminals of the DNA fragment were blunted using Takara DNA Blunting Kit (produced by Takara Shuzo Co., LTD). The resulting DNA fragment was recovered through ethanol precipitation. The DNA was cleaved with EcoRI and subjected to agarose gel electrophoresis, a fragment of approximately 1 kb containing the structural gene of L-proline-3-hydroxylase was extracted from the agarose gel and recovered in a usual manner using Prep-A-Gene (produced by Biorad Co.). The DNA fragment was then dissolved in 10 µl of TE.

On the other hand, 2.4 µg of plasmid pBluescriptII KS(+) DNA was cleaved with ApaI and subjected to agarose gel electrophoresis. The DNA fragment was then recovered from the gel and purified in a usual manner. Using Takara DNA Blunting Kit (produced by Takara Shuzo Co., LTD), the both terminals of the DNA fragment were blunted. The thus-blunted DNA fragment was cleaved with EcoRI and then subjected to ethanol precipitation to obtain an ethanol precipitate. The ethanol precipitate was dissolved in 5 µl of TE.

The above-obtained MluI-EcoRI fragment of approximately 1 kb containing the structural gene of L-proline-3-hydroxylase was ligated to the ApaI-EcoRI-cleaved pBluescriptII KS(+), using a ligation kit (produced by Takara Shuzo Co., LTD).

With the ligated DNA, E. coli XL1-Blue MRF' strain was transformed in a usual manner. The resulting transformant was spread on LB-agar medium containing 50 µg/ml ampicillin and then cultivated thereon overnight at 37°C. A plasmid was extracted from the grown colonies of the transformant in a usual manner, and its structure was identified through digestion with restriction enzyme.

As a result of the above, obtained was plasmid pTH60, into which had been inserted the structural gene of L-proline-3-hydroxylase as fused with the N-terminal amino acid sequence of β-Gal, in the direction same as that for transcription of lac promoter (see Fig. 5). The structure of the amino acid sequence of the fused protein thus constructed hereinabove was such that the N-terminal sequence of β-Gal comprised of 21 amino acids has been fused to the L-proline-3-hydroxylase protein via arginine (22nd) as newly formed at the DNA binding site. However, methionine (GTG) which is the first amino acid of the L-proline-3-hydroxylase protein is translated as valine. The amino acid sequence of the fused protein is indicated by Sequence No. 15.

### (2) pTH70 Plasmid:

pBluescriptII KS(+) DNA (2.4 µg) was cleaved with AccI and subjected to ethanol precipitation to obtain an ethanol precipitate (DNA fragment). The both terminals of the DNA fragment were blunted using Takara DNA Blunting Kit (produced by Takara Shuzo Co., LTD). The resulting DNA fragment was cleaved with EcoRI and then subjected to ethanol precipitation to obtain an ethanol precipitate. The ethanol precipitate was dissolved in 5 µl of TE.

The MluI-EcoRI fragment of approximately 1 kb containing the structural gene of L-proline-3-hydroxylase, which had been obtained in (1) of Example 5, was ligated to the AccI-EcoRI-cleaved pBluescriptII KS(+), using a ligation kit (produced by Takara Shuzo Co., LTD).

With the ligated DNA, E. coli XL1-Blue MRF' strain was transformed in a usual manner. The resulting transformant was spread on LB-agar medium containing 50 µg/ml ampicillin and then cultivated thereon overnight at 37°C. A plasmid was extracted from the grown colonies of the transformant in a usual manner, and its structure was identified through digestion with restriction enzyme.

As a result of the above, obtained was plasmid pTH70, into which had been inserted the structural gene of L-proline-3-hydroxylase as fused with the N-terminal amino acid sequence of β-Gal in the direction same as that for transcription of lac promoter (see Fig. 6). The structure of the amino acid sequence of the fused protein thus constructed hereinabove was such that the N-terminal sequence of β-Gal comprised of 27 amino acids has been fused to the L-proline-3-hydroxylase via arginine (28th) as newly formed at the DNA binding site. However, methionine (GTG) which is the first amino acid of the L-proline-3-hydroxylase protein is translated as valine. The amino acid sequence of the fused protein is indicated by Sequence No. 16.

### (3) pTH80 Plasmid:

Four mirograms of pTH40 DNA was cleaved with MluI and subjected to ethanol precipitation to obtain an ethanol precipitate. The ethanol precipitate (DNA fragment) was dissolved in 36 µl of TE. The both terminals of the DNA fragment were blunted using Takara DNA Blunting Kit (produced by Takara Shuzo Co., LTD). The resulting DNA fragment cleaved with SacII and then subjected to agarose gel electrophoresis. After the electrophoresis, a fragment of approximately 0.95 kb containing the structural gene of L-proline-3-hydroxylase was extracted from the gel and recovered in a usual manner using Prep-A-Gene (produced by Biorad Co.). The thus-recovered fragment was then dissolved in 10 µl of TE.

On the other hand, 2.4 µg of plasmid pBluescriptII KS(+) DNA was cleaved with PstI and subjected to ethanol precipitation to obtain an ethanol precipitate (DNA fragment). This DNA fragment was dissolved in 36 µl of TE, and the both terminals of the DNA fragment were blunted using Takara DNA Blunting Kit (produced by Takara Shuzo Co., LTD). The resulting DNA fragment was then cleaved with SacII and subjected to ethanol precipitation to obtain an ethanol precipitate (DNA fragment). The DNA fragment was then dissolved in 5 µl of TE.

The MluI-SacII fragment of approximately 1 kb containing the structural gene of L-proline-3-hydroxylase, which had been obtained in the above, was ligated to the PstI-SacII-cleaved pBluescriptII KS(+), using a ligation kit (produced by Takara Shuzo Co., LTD).

With the ligated DNA, E. coli XL1-Blue MRF' strain was transformed in a usual manner. The resulting transformant was spread on LB-agar medium containing 50 µg/ml ampicillin and then cultivated thereon overnight at 37°C. A plasmid was extracted from the grown colonies of the transformant in a usual manner, and its structure was identified through digestion with restriction enzyme.

As a result of the above, obtained was plasmid pTH80, into which had been inserted the structural gene of L-proline-3-hydroxylase as fused with the N-terminal amino acid sequence of β-Gal in the direction same as that for transcription of lac promoter (see Fig. 7). The structure of the amino acid sequence of the fused protein thus constructed hereinabove was such that the N-terminal sequence of β-Gal comprised of 37 amino acids has been fused to the L-proline-3-hydroxylase protein via arginine (38th) as newly formed at the DNA binding site. However, methionine (GTG) which is the first amino acid of the L-proline-3-hydroxylase protein is translated as valine. The amino acid sequence of the fused protein is indicated by Sequence No. 17.

### Example 6: Production of L-proline-3-hydroxylase by Transformant Having Expression Plasmid for Fused Protein :

E. coli ATCC12435 was transformed with any one of plasmids pTH60, pTH70 and pTH80 as obtained in Example 5. In the same manner as in Example 4, the resulting transformant was cultivated, and the productivity of L-proline-3-hydroxylase by the transformant cells was examined.

As shown in Table 3 below, the transformant produced L-proline-3-hydroxylase in an amount of 46 to 121 times per cell in comparison to Streptomyces sp. TH1 strain which had been used as

**Table 3**

| L-proline-3-hydroxylase Activity Produced by Transformants | | |
|---|---|---|
| Strain | Cell Activity¹⁾ | Relative Activity²⁾ |
| E. coli ATCC12435/pTH60 | 5.19 | 91.1 |
| E. coli ATCC12435/pTH70 | 6.90 | 121.1 |
| E. coli ATCC12435/pTH80 | 2.63 | 46.1 |
| E. coli ATCC12435/pBluescriptII KS (+) | Not detected. | - |
| Streptomyces sp. TH1³⁾ | 0.057 | 1.0 |

| | | |
|---|---|---|
| 1) Cell activity indicates the enzymatic activity per mg of wet cells (U/mg wet cells). One U indicates the enzymatic activity of producing 1 nmol of cis-3-hydroxy-L-proline per minute (nmol/min). | | |
| 2) Relative activity is based on the enzymatic activity produced by Streptomyces sp. TH1 strain of being 1 (one). | | |
| 3) This is referred to in Reference Example 2. | | |

### Example 7: Production of cis-3-hydroxy-L-proline by Transformant:

### (1) Production of cis-3-hydroxy-L-proline by Transformant E. coli ATCC12435/pTH70:

cis-3-hydroxy-L-proline was produced using the transformant E. coli ATCC12935/pTH70 as obtained in Example 6.

Precisely, the transformant of E. coli ATCC12435/pTH70 was inoculated in 3 ml of a TB medium (comprising 12 g of bactotryptone, 24 g of bactoyeast extract, 4 g of glycerol, 2.3 g of KH₂PO₄ and 12.5 g of dipotassium phosphate in one liter of distilled water and sterilized at 120°C for 20 minutes) containing 100 µg/ml ampicillin, and cultivated therein at 30°C for 16 hours by shaking culture. The resulting culture was centrifuged, and the amount of cis-3-hydroxy-L-proline in the supernatant thus separated was quantitatively determined.

As a result, 620 µM (81.1 mg/liter) of cis-3-hydroxy-L-proline was formed in the supernatant of the culture of E. coli ATCC12435/pTH70.

On the other hand, cis-3-hydroxy-L-proline was not detected in the supernatant of the culture of the E. coli ATCC12435. which had been used as the host.

### (2) Production of Cis-3-hydroxy-L-proline by Transformant E. coli ATCC12435/pTH70:

The transformant of E. coli ATCC12435/pTH70 was inoculated in 50 ml of a Med4 medium containing 100 µg/ml ampicillin, and cultivated therein at 30°C for 16 hours by shaking.

The culture was used as a seed culture, which was inoculated in a 5-liter jar fermenter containing therein 2 liters of a Med6 medium. 200 mM L-proline and 100 µg/ml ampicillin were added thereto, and the transformant was cultivated in the fermenter under the condition of 400 rpm and 1vvm, at 30°C.

During the cultivation, glucose and L-proline were suitably added to the medium in such a manner that glucose was always present in the medium and L-proline could be at about 50 mM therein, and the lowermost limit of the pH of the medium was controlled at 6.5 by adding NH₄OH to the medium.

The culture was centrifuged, and the amount of cis-3-hydroxy-L-proline in the supernatant separated was quantitatively determined. Seventy two hours after the start of the incubation, 115 mM (15 g/liter) cis-3-hydroxy-L-proline was produced and accumulated in the supernatant of the culture of E. coli ATCC12435/pTH70.

On the other hand, free cis-3-hydroxy-L-proline was not detected in the supernatant of the culture of E. coli ATCC12435 which had been used as the host.

### Example 8: Conversion of L-proline into Cis-3-hydroxy-L-proline with Transformant cells:

Using the transformant E. coli ATCC12435/pTH70 as obtained in Example 6, L-proline was converted into cis-3-hydroxy-L-proline.

Precisely, the transformant was inoculated in 10 ml of an LB medium containing 50 µg/ml ampicillin and cultivated therein overnight at 30°C by shaking. Then 7 ml of the culture was centrifuged to collect the cells (wet cells). If desired, the cells were frozen and stored at -20°C and thawed before use.

After 10 %(w/v) of the wet cells were added to 500 µl of a reaction mixture [comprising 24 mM L-proline, 24 mM 2-ketoglutaric acid, 4 mM ferrous sulfate and 8 mM L-ascorbic acid in 200 mM TES buffer (pH 7.5)], reaction was conducted at 35°C for 60 minutes. The amount of cis-3-hydroxy-L-proline as formed in the reaction mixture was quantitatively determined. As a result, 17.7 mM (2.3 g/liter) of cis-3-hydroxy-L-proline was formed in the reaction mixture.

### Reference Example 1: Isolation and Purification of L-proline-3-hydroxylase:

### (1) Preparation of Frozen Cells of Streptomyces sp. TH1:

Ten milliliters of an SR3 medium (comprising 1.0 % glucose, 1.0 % soluble starch, 0.5 % yeast extract, 0.5 % tryptone, 0.3 % meat extract and 0.05 % magnesium phosphate and adjusted at pH 7.2 with 6 N NaOH) was put into a test tube and sterilized at 120°C for 20 minutes. One loopful of cells of Streptomyces sp. TH1, that had grown in HT-agar plate medium (comprising 1% soluble starch, 0.2% NZ amine, 0.1% yeast extract, 0.1% meat extract and 1.5% agar, adjusted to pH 7.2 with 6N NaOH, and sterilized at 120°C for 20 minutes) was inoculated into the above-mentioned SR3 medium in each test tube and cultivated at 28°C for 2 days by shaking. The resulting culture was used as the seed culture in the following step.

Df3 medium comprising 5% soluble starch, 3% corn steep liquor, 0.05% potassium dihydrogen phosphate, 0.05% magnesium sulfate 7 hydrate and 0.5% calcium carbonate, and adjusted to pH 7.0 with 6N NaOH, was put in 5 liter-jar fermenters in an amount of 2 liters each and sterilized at 120°C for 20 minutes. The above-mentioned seed culture was inoculated in the DF3 medium in each jar fermenter under germ-free condition and cultivated under the condition of 700 rpm and 1 vvm, at 28°C for 2 days. During the cultivation, the pH of the medium was not adjusted. The thus-obtained culture was subjected to centrifugation at 15,000 x g for 10 minutes at 4°C and 75g of the wet cells thus separated were obtained per liter of the culture. The wet cells were washed with a physiological saline at 4°C and then recentrifuged. The thus-obtained wet cells were frozen and stored at -80°C before use.

### (2) Preparation of Cell-free Extract:

Thirty grams of the wet cells obtained in (1) was suspended in 200 ml of Buffer (A) [20 mM piperazine buffer, pH was adjusted to 5.3 with 6N HCl, containing 1 mM dithiothreitol (DTT), 0.2 mM EDTA, 0.1% (v/v) Tween 20 and 10% (v/v) glycerol] while cooling with ice. The cells in the resulting suspension was disrupted by means of an ultrasonic cell disruptor while cooling with ice. The thus-disrupted cell suspension was subjected to centrifugation at 30,000 x g at 4°C for 30 minutes to separate a supernatant.

The subsequent operations were conducted under cooling with ice at a temperature of 4°C or lower.

### (3) Isolation and Purification by Column Chromatography:

### (3)-1 Acid Treatment

The pH of the supernatant obtained in the previous step was adjusted at 4.5 with 6 N HCl, and the precipitate formed was removed therefrom through centrifugation as conducted at 15,000 g for 30 minutes to obtain a supernatant.

### (3)-2 Resource Q Column Chromatography (I):

The supernatant obtained in the previous step was passed through a RESOURCE™ Q column (6 ml; produced by Pharmacia Co.) that had been equilibrated with Buffer (A). The column was washed with Buffer (A), and the fraction containing the desired enzyme was eluted with Buffer (A) having a linear concentration gradient of NaCl of 0 to 0.3M.

### (3)-3 Resource Q Column Chromatography (II):

The active fraction obtained in the previous step was diluted 3-fold with Buffer (B) [20 mM TES buffer (pH 7.5) containing 1 mM DTT, 0.2 mM EDTA and 10% (v/v) of glycerol] and was passed through a RESOURCE™ Q column (1 ml; produced by Pharmacia Co.) that had been equilibrated with Buffer (B). After the column was washed with Buffer (B), the fraction containing the enzyme was eluted with Buffer (B) having a linear concentration gradient of NaCl of 0 to 0.3 M.

### (3)-4 Phenyl Sepharose Column Chromatography:

NaCl was added to the active fraction obtained in the previous step until an NaCl concentration was 2M. The mixture was applied to a Phenyl Sepharose column (1 ml; Phenyl Sepharose HP HiLoad) that had been equilibrated with Buffer (B) containing 2M NaCl. The column was washed with Buffer (B) containing 2M NaCl, and the fraction containing the desired enzyme was eluted with Buffer (B) containing 0.1% (v/v) of Tween 20.

### (3)-5 Resource Q Column Chromatography (III):

The active fraction obtained in the previous step was de-salted using a PD-10 column, and the resulting mixture was passed through a RESOURCE^{TH} Q column (1 ml; produced by Pharmacia Co.) that had been equilibrated with Buffer (A). After the column was washed with Buffer (A), the fraction containing the desired enzyme was eluted with Buffer (A) having a linear concentration gradient of NaCl of 0 to 0.3 M.

### (3)-6 Resource Q Column Chromatography (IV):

The active fraction obtained in the previous step was diluted 3-fold with Buffer (B) containing 0.1% (v/v) Tween 20 and passed through a RESOURCE^{TH} Q column (1 ml; produced by Pharmacia Co.) that had been equilibrated with Buffer (B). The column was washed with Buffer (B), and the fraction containing the desired enzyme was eluted with Buffer B having a linear concentration gradient of NaCl of 0 to 0.3M.

The foregoing steps for the isolation and purification of the L-proline-3-hydroxylase are summarized in Table 4.

**Table 4**

| Summary of Isolation and Purification of L-proline-3-Hydroxylase | | | | |
|---|---|---|---|---|
| Fraction | Total Protein (mg) | Total Activity (U) | Relative Activity (U/mg protein) | Yield (%) |
| Cell-free Extract | 542 | 3540 | 6.5 | 100 |
| Acid-treatment Supernatant, pH 4.5 | 106 | 1800 | 17 | 56 |
| Resource Q(I), pH 5.3 | 7.5 | 1553 | 207 | 44 |
| Resource Q(II), pH 7.5 | 3.3 | 1036 | 306 | 29 |
| Phenyl-Sepharose | 0.43 | 188 | 437 | 5.3 |
| Resource Q(III), pH 5.3 | 0.16 | 90.5 | 566 | 2.6 |
| Resource Q(IV), pH 7.5 | 0.035 | 60.3 | 1723 | 1.7 |

### Reference Example 2: Production of L-proline-3-hydroxylase by Streptomyces sp.:

SR3 medium was put into a test tube in an amount of 10 ml and sterilized at 120°C for 20 minutes. One loopful of cells of Streptomyces sp. TH1, that had grown in HT-agar plate medium, was inoculated into the above-mentioned SR3 medium in each test tube and cultivated at 28°C for 2 days by shaking. The resulting culture was used as the seed culture in the following step.

Df3 medium was put into a test tube in an amount of 10 ml each and sterilized at 120°C for 20 minutes. The above-mentioned seed culture was inoculated in the Df3 medium in each test tube under germ-free condition and cultivated at 28°C for 2 days with shaking. The thus-obtained culture was subjected to centrifugation at 8,000 rpm for 10 minutes at 4°C. The cells thus separated were suspended in 80 mM TES (N-tris(hydroxymethyl)methyl-2-aminoethanesulfonic acid)buffer (pH 7.5), washed and then centrifuged to separate the wet cells.

One gram of the thus-obtained wet cells was suspended in 10 ml of a reaction mixture [prepared by adding 1.4% (v/v) of Nymeen solution (prepared by dissolving 4g of Nymeen S-215 (produced by Nippon Oils & Fats Co.) in 10 ml of xylene) to 100 mM TES buffer (pH 7.5) containing 5 mM L-proline, 5 mM α-ketoglutaric acid, 5 mM L-ascorbic acid and 1 mM ferrous sulfate] and the mixture was allowed to stand at 30°C for 30 minutes. After the reaction, the cells were removed from the reaction mixture by centrifugation, and the amount of cis-3-hydroxy-L-proline formed in the supernatant was quantitatively determined.

The results are shown in Table 1.

### INDUSTRIAL APPLICABILITY OF THE INVENTION

According to the present invention, there are provided industrial methods for producing cis-3-hydroxy-L-proline which is useful as a starting compound for medicines and an additive to foods, genes which code for a protein having the enzymatic activity of hydroxylating the 3-position of L-proline and which are useful for the above-mentioned process, transformants containing the gene, methods for producing L-proline-3-hydroxylases using the transformants and L-proline-3-hydroxylases.

### SEQUENCE LISTING

SEQUENCE NO. :1
   SEQUENCE LENGTH: 290
   SEQUENCE TYPE: amino acid
   TOPOLOGY: linear
   MOLECULE TYPE: protein
   ORIGINAL SOURCE
      ORGANISM: Streptomyces sp.
      STRAIN: TH1
   SEQUENCE: 
Met Arg Ser His Ile Leu Gly Arg Ile Glu Leu Asp Gln Glu Arg Leu
1               5                   10                  15
Gly Arg Asp Leu Glu Tyr Leu Ala Thr Val Pro Thr Val Glu Glu Glu
            20                  25                  30
Tyr Asp Glu Phe Ser Asn Gly Phe Trp Lys Asn Ile Pro Leu Tyr Asn
        35                  40                  45
Ala Ser Gly Gly Ser Glu Asp Arg Leu Tyr Arg Asp Leu Glu Gly Ser
    50                  55                  60
Pro Ala Gln Pro Thr Lys His Ala Glu Gln Val Pro Tyr Leu Asn Glu
65                  70                  75                  80
Ile Ile Thr Thr Val Tyr Asn Gly Glu Arg Leu Gln Met Ala Arg Thr
                85                  90                  95
Arg Asn Leu Lys Asn Ala Val Val Ile Pro His Arg Asp Phe Val Glu
            100                 105                 110
Leu Asp Arg Glu Leu Asp Gln Tyr Phe Arg Thr His Leu Met Leu Glu
        115                 120                 125
Asp Ser Pro Leu Ala Phe His Ser Asp Asp Asp Thr Val Ile His Met
    130                 135                 140
Arg Ala Gly Glu Ile Trp Phe Leu Asp Ala Ala Ala Val His Ser Ala
145                 150                 155                 160
Val Asn Phe Ala Glu Phe Ser Arg Gln Ser Leu Cys Val Asp Leu Ala
                165                 170                 175
Phe Asp Gly Ala Phe Asp Glu Lys Glu Ala Phe Ala Asp Ala Thr Val
            180                 185                 190
Tyr Ala Pro Asn Leu Ser Pro Asp Val Arg Glu Arg Lys Pro Phe Thr
        195                 200                 205
Lys Glu Arg Glu Ala Gly Ile Leu Ala Leu Ser Gly Val Ile Gly Arg
    210                 215                 220
Glu Asn Phe Arg Asp Ile Leu Phe Leu Leu Ser Lys Val His Tyr Thr
225                 230                 235                 240
Tyr Asp Val His Pro Gly Glu Thr Phe Glu Trp Leu Val Ser Val Ser
                245                 250                 255
Lys Gly Ala Gly Asp Asp Lys Met Val Glu Lys Ala Glu Arg Ile Arg
            260                 265                 270
Asp Phe Ala Ile Gly Ala Arg Ala Leu Gly Glu Arg Phe Ser Leu Thr
        275                 280                 285
Thr Trp
    290
SEQUENCE NO. :2
   SEQUENCE LENGTH: 290
   SEQUENCE TYPE: amino acid
   TOPOLOGY: linear
   MOLECULE TYPE: protein
   ORIGINAL SOURCE
      ORGANISM: Streptomyces sp.
      STRAIN: TH1
   SEQUENCE: 
Met Arg Ser His Ile Leu Gly Lys Ile Glu Leu Asp Gln Thr Arg Leu
1               5                   10                  15
Ala Pro Asp Leu Ala Tyr Leu Ala Ala Val Pro Thr Val Glu Glu Glu
            20                  25                  30
Tyr Asp Glu Phe Ser Asn Gly Phe Trp Lys His Val Pro Leu Trp Asn
        35                  40                  45
Ala Ser Gly Asp Ser Glu Asp Arg Leu Tyr Arg Asp Leu Lys Asp Ala
    50                  55                  60
Ala Ala Gln Pro Thr Ala His Val Glu His Val Pro Tyr Leu Lys Glu
65                  70                  75                  80
Ile Val Thr Thr Val Phe Asp Gly Thr His Leu Gln Met Ala Arg Ser
                85                  90                  95
Arg Asn Leu Lys Asn Ala Ile Val Ile Pro His Arg Asp Phe Val Glu
            100                 105                 110
Leu Asp Arg Glu Val Asp Arg Tyr Phe Arg Thr Phe Met Val Leu Glu
        115                 120                 125
Asp Ser Pro Leu Ala Phe His Ser Asn Glu Asp Thr Val Ile His Met
    130                 135                 140
Arg Pro Gly Glu Ile Trp Phe Leu Asp Ala Ala Thr Val His Ser Ala
145                 150                 155                 160
Val Asn Phe Ser Glu Ile Ser Arg Gln Ser Leu Cys Val Asp Phe Ala
                165                 170                 175
Phe Asp Gly Pro Phe Asp Glu Lys Glu Ile Phe Ala Asp Ala Thr Leu
            180                 185                 190
Tyr Ala Pro Gly Ser Thr Pro Asp Leu Pro Glu Arg Arg Pro Phe Thr
        195                 200                 205
Ala Glu His Arg Arg Arg Ile Leu Ser Leu Gly Gln Val Ile Glu Arg
    210                 215                 220
Glu Asn Phe Arg Asp Ile Leu Phe Leu Leu Ser Lys Val His Tyr Lys
225                 230                 235                 240
Tyr Asp Val His Pro Ser Glu Thr Tyr Asp Trp Leu Ile Glu Ile Ser
                245                 250                 255
Lys Gln Ala Gly Asp Glu Lys Met Val Val Lys Ala Glu Gln Ile Arg
            260                 265                 270
Asp Phe Ala Val Glu Ala Arg Ala Leu Ser Glu Arg Phe Ser Leu Thr
        275                 280                 285
Ser Trp
    290
SEQUENCE NO. :3
   SEQUENCE LENGTH: 870
   SEQUENCE TYPE: nucleic acid
   STRANDEDNESS: double
   MOLECULE TYPE: genomic DNA
   ORIGINAL SOURCE
      ORGANISM: Streptomyces sp.
      STRAIN: TH1
   SEQUENCE: 
GTGCGCTCGC ACATACTCGG CCGCATTGAA CTCGACCAGG AACGCTTAGG CAGGGACCTC 60
GAATATCTCG CCACGGTGCC CACCGTGGAA GAGGAGTACG ACGAGTTCAG CAACGGGTTC 120
TGGAAGAACA TCCCGCTGTA CAACGCGAGC GGCGGCAGCG AGGACCGGCT GTACCGCGAC 180
CTCGAGGGCT CACCGGCGCA GCCCACCAAA CACGCCGAGC AGGTTCCGTA CCTCAACGAG 240
 
ATCATCACCA CGGTCTACAA CGGCGAGCGG CTCCAGATGG CGCGTACGCG GAACCTGAAG 300
AACGCCGTCG TCATCCCGCA CCGCGACTTC GTGGAGCTCG ACCGCGAACT CGACCAGTAC 360
TTCCGCACCC ATTTGATGCT TGAGGACAGC CCGCTGGCCT TCCACTCGGA CGACGACACC 420
GTCATCCACA TGCGGGCCGG CGAGATCTGG TTCCTCGACG CGGCCGCCGT CCACTCGGCC 480
GTCAACTTCG CCGAGTTCAG CAGGCAGTCG CTCTGCGTCG ACCTCGCCTT CGACGGCGCG 540
TTCGACGAGA AGGAAGCCTT CGCGGACGCC ACGGTCTACG CCCCGAACCT CAGCCCCGAC 600
GTCCGCGAAC GCAAGCCGTT CACCAAGGAG CGGGAGGCCG GGATCCTCGC CCTGTCCGGC 660
GTGATCGGAC GCGAGAACTT CCGGGACATC CTCTTTCTGC TGTCCAAGGT CCACTACACC 720
TACGACGTCC ATCCGGGTGA AACCTTCGAG TGGCTCGTGA GCGTCTCCAA GGGTGCGGGA 780
GACGACAAGA TGGTGGAGAA GGCCGAGCGG ATCAGGGACT TCGCCATCGG CGCACGGGCA 840
CTCGGCGAGC GTTTCTCGCT GACCACCTGG 870
SEQUENCE NO.:4
   SEQUENCE LENGTH: 870
   SEQUENCE TYPE: nucleic acid
   STRANDEDNESS: double
   MOLECULE TYPE: genomic DNA
   ORIGINAL SOURCE
      ORGANISM: Streptomyces sp.
      STRAIN: TH1
   SEQUENCE: 
ATGCGCTCGC ACATCCTTGG CAAGATCGAA CTCGATCAGA CCCGGTTGGC GCCGGATCTC 60
GCATACCTCG CCGCAGTTCC GACCGTGGAG GAGGAGTACG ACGAGTTCAG CAACGGGTTC 120
TGGAAGCACG TGCCCCTGTG GAACGCCTCC GGGGACAGCG AGGACCGGCT CTACCGCGAC 180
CTCAAGGACG CCGCCGCACA GCCGACCGCG CACGTGGAGC ACGTCCCCTA CCTCAAGGAG 240
ATCGTGACCA CGGTCTTCGA CGGCACGCAC CTGCAGATGG CGCGGAGCCG GAACCTGAAG 300
AACGCCATCG TCATCCCGCA CCGCGACTTC GTGGAGCTGG ACCGCGAAGT CGACCGGTAC 360
TTCCGCACGT TCATGGTGCT GGAGGACAGC CCGCTCGCCT TCCACTCGAA CGAGGACACC 420
GTCATCCACA TGCGCCCGGG CGAAATATGG TTCCTGGACG CGGCGACGGT GCACTCCGCG 480
GTCAACTTCT CGGAAATCAG CCGTCAGTCC CTGTGCGTCG ACTTCGCCTT CGACGGTCCC 540
TTCGACGAGA AGGAGATCTT CGCGGACGCC ACCCTCTACG CTCCGGGCTC CACGCCCGAC 600
CTGCCCGAGC GCCGCCCCTT CACCGCGGAG CACCGGCGGC GGATCCTCTC CCTGGGCCAG 660
GTGATCGAGC GGGAGAACTT CCGGGACATT CTGTTCCTGC TGTCCAAGGT GCACTACAAG 720
TACGACGTGC ACCCCAGCGA GACGTACGAC TGGCTGATCG AGATCTCGAA ACAGGCCGGC 780
GACGAGAAGA TGGTCGTGAA GGCGGAGCAG ATCAGGGACT TCGCCGTCGA GGCCCGCGCC 840
CTGAGCGAGC GCTTCTCCCT GACCTCCTGG 870
SEQUENCE NO. :5
   SEQUENCE LENGTH: 29
   SEQUENCE TYPE: amino acid
   TOPOLOGY: linear
   MOLECULE TYPE: peptide
   FRAGMENT TYPE: N-terminal fragment
   ORIGINAL SOURCE
      ORGANISM: Streptomyces sp.
      STRAIN: TH1
   SEQUENCE: 
Met Arg Ser His Ile Leu Gly Arg Ile Glu Leu Asp Gln Glu Arg Leu
1               5                   10                  15
Gly Arg Asp Leu Glu Tyr Leu Ala Thr Val Pro Thr Val
            20                  25
SEQUENCE NO. :6
   SEQUENCE LENGTH: 25
   SEQUENCE TYPE: amino acid
   TOPOLOGY: linear
   MOLECULE TYPE: peptide
   FRAGMENT TYPE: internal fragment
   ORIGINAL SOURCE
      ORGANISM: Streptomyces sp.
      STRAIN: TH1
   SEQUENCE: 
Glu Ala Phe Ala Asp Ala Thr Val Tyr Ala Pro Asn Leu Ser Pro Asp
1               5                   10                  15
Val Arg Glu Arg Lys Pro Phe Thr Lys
            20                  25
SEQUENCE NO.:7
   SEQUENCE LENGTH: 25
   SEQUENCE TYPE: amino acid
   TOPOLOGY: linear
   MOLECULE TYPE: peptide
   FRAGMENT TYPE: internal fragment
   ORIGINAL SOURCE
      ORGANISM: Streptomyces sp.
      STRAIN: TH1
   SEQUENCE: 
Asn Ile Pro Leu Tyr Asn Ala Ser Gly Gly Ser Glu Asp Arg Leu Tyr
1               5                   10                  15
Arg Asp Leu Glu Gly Ser Pro Ala Gln
            20                  25
SEQUENCE NO.:8
   SEQUENCE LENGTH: 17
   SEQUENCE TYPE: nucleic acid
   STRANDEDNESS: single
   MOLECULE TYPE: other nucleic acid, synthetic DNA
   SEQUENCE: ATGTGYTCNC AYATHYT 17
SEQUENCE NO. :9
   SEQUENCE LENGTH: 14
   SEQUENCE TYPE: nucleic acid
   STRANDEDNESS: single
   MOLECULE TYPE: other nucleic acid, synthetic DNA
   SEQUENCE: GTNGGNACNG TWGC 14
SEQUENCE NO.:10
   SEQUENCE LENGTH: 14
   SEQUENCE TYPE: nucleic acid
   STRANDEDNESS: single
   MOLECULE TYPE: other nucleic acid, synthetic DNA
   SEQUENCE: GTNGCNAGRT AYTC 14
SEQUENCE NO. :11
   SEQUENCE LENGTH: 74
   SEQUENCE TYPE: nucleic acid
   STRANDEDNESS: double
   MOLECULE TYPE: other nucleic acid, synthetic DNA
   SEQUENCE: 
ATG TGT TCG CAT ATA CTC GGC CGC ATT GAA CTC GAC CAG GAA CGC TTA 48
Met Cys Ser His Ile Leu Gly Arg Ile Glu Leu Asp Gln Glu Arg Leu
1               5                   10                  15
GGC AGG GAC CTC GAA TAC CTC GCC AC 74
Gly Arg Asp Leu Glu Tyr Leu Ala
            20
SEQUENCE NO.:12
   SEQUENCE LENGTH: 74
   SEQUENCE TYPE: nucleic acid
   STRANDEDNESS: double
   MOLECULE TYPE: other nucleic acid, synthetic DNA
   SEQUENCE: 
ATG TGC TCG CAT ATC CTT GGC AAG ATC GAA CTC GAT CAG ACC CGG TTG 48
Met Cys Ser His Ile Leu Gly Lys Ile Glu Leu Asp Gln Thr Arg Leu
1               5                   10                  15
GCG CCG GAT CTC GAG TAC CTC GCC AC 74
Ala Pro Asp Leu Glu Tyr Leu Ala
            20
SEQUENCE NO. :13
   SEQUENCE LENGTH: 1081
   SEQUENCE TYPE: nucleic acid
   STRANDEDNESS: double
   MOLECULE TYPE: genomic DNA
   ORIGINAL SOURCE
      ORGANISM: Streptomyces sp.
      STRAIN: TH1
   SEQUENCE: 
GGTACCCATT CCGTCACCGA AAGCTGAACT CGACACCAGG AGGACGACGC GTGCGCTCGC 60
ACATACTCGG CCGCATTGAA CTCGACCAGG AACGCTTAGG CAGGGACCTC GAATATCTCG 120
CCACGGTGCC CACCGTGGAA GAGGAGTACG ACGAGTTCAG CAACGGGTTC TGGAAGAACA 180
TCCCGCTGTA CAACGCGAGC GGCGGCAGCG AGGACCGGCT GTACCGCGAC CTCGAGGGCT 240
CACCGGCGCA GCCCACCAAA CACGCCGAGC AGGTTCCGTA CCTCAACGAG ATCATCACCA 300
CGGTCTACAA CGGCGAGCGG CTCCAGATGG CGCGTACGCG GAACCTGAAG AACGCCGTCG 360
TCATCCCGCA CCGCGACTTC GTGGAGCTCG ACCGCGAACT CGACCAGTAC TTCCGCACCC 420
ATTTGATGCT TGAGGACAGC CCGCTGGCCT TCCACTCGGA CGACGACACC GTCATCCACA 480
TGCGGGCCGG CGAGATCTGG TTCCTCGACG CGGCCGCCGT CCACTCGGCC GTCAACTTCG 540
CCGAGTTCAG CAGGCAGTCG CTCTGCGTCG ACCTCGCCTT CGACGGCGCG TTCGACGAGA 600
AGGAAGCCTT CGCGGACGCC ACGGTCTACG CCCCGAACCT CAGCCCCGAC GTCCGCGAAC 660
GCAAGCCGTT CACCAAGGAG CGGGAGGCCG GGATCCTCGC CCTGTCCGGC GTGATCGGAC 720
GCGAGAACTT CCGGGACATC CTCTTTCTGC TGTCCAAGGT CCACTACACC TACGACGTCC 780
ATCCGGGTGA AACCTTCGAG TGGCTCGTGA GCGTCTCCAA GGGTGCGGGA GACGACAAGA 840
TGGTGGAGAA GGCCGAGCGG ATCAGGGACT TCGCCATCGG CGCACGGGCA CTCGGCGAGC 900
GTTTCTCGCT GACCACCTGG TAGACGGCCG AAGAACGGGG CCCGGGGGAA CAGTGATCGA 960
GGAGATACTT CCCGTCGACG TGATGTCCGC GGAGGCGTTC GACGACGATG CGGACATCCA 1020
GCTCTTCGCC GAGGAACGCG CGGCCGTCGC CGATGCCGTA CCGCGGCGCC GACGGGAATT 1080
C
SEQUENCE NO.:14
   SEQUENCE LENGTH: 1826
   SEQUENCE TYPE: nucleic acid
   STRANDEDNESS: double
   MOLECULE TYPE: genomic DNA
   ORIGINAL SOURCE
      ORGANISM: Streptomyces sp.
      STRAIN: TH1
   SEQUENCE: 
TCGCGACCTG GAGGGTCGGA TGCGCTCGCA CATCCTTGGC AAGATCGAAC TCGATCAGAC 60
CCGGTTGGCG CCGGATCTCG CATACCTCGC CGCAGTTCCG ACCGTGGAGG AGGAGTACGA 120
CGAGTTCAGC AACGGGTTCT GGAAGCACGT GCCCCTGTGG AACGCCTCCG GGGACAGCGA 180
GGACCGGCTC TACCGCGACC TCAAGGACGC CGCCGCACAG CCGACCGCGC ACGTGGAGCA 240
CGTCCCCTAC CTCAAGGAGA TCGTGACCAC GGTCTTCGAC GGCACGCACC TGCAGATGGC 300
GCGGAGCCGG AACCTGAAGA ACGCCATCGT CATCCCGCAC CGCGACTTCG TGGAGCTGGA 360
CCGCGAAGTC GACCGGTACT TCCGCACGTT CATGGTGCTG GAGGACAGCC CGCTCGCCTT 420
CCACTCGAAC GAGGACACCG TCATCCACAT GCGCCCGGGC GAAATATGGT TCCTGGACGC 480
GGCGACGGTG CACTCCGCGG TCAACTTCTC GGAAATCAGC CGTCAGTCCC TGTGCGTCGA 540
CTTCGCCTTC GACGGTCCCT TCGACGAGAA GGAGATCTTC GCGGACGCCA CCCTCTACGC 600
TCCGGGCTCC ACGCCCGACC TGCCCGAGCG CCGCCCCTTC ACCGCGGAGC ACCGGCGGCG 660
GATCCTCTCC CTGGGCCAGG TGATCGAGCG GGAGAACTTC CGGGACATTC TGTTCCTGCT 720
GTCCAAGGTG CACTACAAGT ACGACGTGCA CCCCAGCGAG ACGTACGACT GGCTGATCGA 780
GATCTCGAAA CAGGCCGGCG ACGAGAAGAT GGTCGTGAAG GCGGAGCAGA TCAGGGACTT 840
CGCCGTCGAG GCCCGCGCCC TGAGCGAGCG CTTCTCCCTG ACCTCCTGGT AATGGCGCGA 900
CCTCAGCCCG CGTGACAACA GTTCGGCCCC GGTGGCGCGG NGCGTCCCGG ACGCGCGCCG 960
GNCGCTACCG GACACCGGGG CACGTAGGTG GTGTACCGNC TGNCGGTCTC CGGCAGTCAG 1020
TTGCTGGTTC GACTCCAGCT GCCCCGACCG CCTCGCGCTC GGACGCGACC CCGGGCACCT 1080
CCGNGGGCAC CGCGTTCGCA TCACGGACAC CCCACCCACA CCAGCCGGGC CCACCCGAGG 1140
AGCCAGTTCA CATGTCCAGT GACATACACG CGGCGGCATC CAGACCGATT CCGCCGAAGC 1200
CCGCCGCATC GACGGAGGTC GCGCCGCGCA CGCTGGTCAG CCGGCTGCCC TCGCTGACCG 1260
GCCTGCGCTT CCCGGCGGCG TTCATCGTGT TCCTCTTCCA CGCCTCGCTG CCGTTTCCCG 1320
CGGTGCGCCT GTTCGCCGAC GACGGGGTGG AACACCGCTA CGGGTGGGCC CTCGGCCCGA 1380
GCGGCGCACT CGGCGTGACG TTCTTCTTCG CGCTCAGCGG ATTCGTGCTG ACGTGGTCGG 1440
CTCCCGCCGG CGACACCGCA CCGTCCTTCT GGCGCCGGCG CTTCGTCAAG ATCCTGCCCA 1500
ACTACGTCGT CGCGTGGGTC CTGGCGATGG TGCTGTACGC GGCCGCGACG CCCGTCCTGC 1560
CCGCGCTCGG CGCCCTTTTC ATGCTCCAGG TGTGGACGCC GTACTTCACC GAGCACCTCC 1620
CGGTGAACCC ACCGAGCTGG TCGCTGNCCG TGGAAGCCGT CTTCTATCTG GCCTTCCCGT 1680
TCCTGCTGGC CGGGATCAGA CGGATACCGG CCGCCCGGCT GAAGTACTGG ATCGCCGGCA 1740
CGGTGGCCGC CGTCTTCGCC ACGCCGCTGA TCACCTACCT CCTGGTGCCG GCGGGCCCGC 1800
ACGTGATGCC GGGCACGGGC GGTACC 1826
SEQUENCE NO. :15
   SEQUENCE LENGTH: 312
   SEQUENCE TYPE: amino acid
   TOPOLOGY: linear
   MOLECULE TYPE: protein
   ORIGINAL SOURCE:
      ORGANISM: Streptomyces sp.
      STRAIN: TH1
   SEQUENCE FEATURE:
      NAME/KEY: peptide
      location: 23 to 312
      IDENTIFICATION METHOD: S
   ORIGINAL SOURCE:
      ORGANISM: Escherichia coli
   IMMEDIATE SOURCE: pBluescriptIIKS+
   SEQUENCE FEATURE:
      NAME/KEY: peptide
      location: 1 to 21
      IDENTIFICATION METHOD: S
   SEQUENCE: 
Met Thr Met Ile Thr Pro Ser Ala Gln Leu Thr Leu Thr Lys Gly Asn
1               5                   10                  15
Lys Ser Trp Val Pro Arg Val Arg Ser His Ile Leu Gly Arg Ile Glu
            20                  25                  30
Leu Asp Gln Glu Arg Leu Gly Arg Asp Leu Glu Tyr Leu Ala Thr Val
        35                  40                  45
Pro Thr Val Glu Glu Glu Tyr Asp Glu Phe Ser Asn Gly Phe Trp Lys
    50                  55                  60
Asn Ile Pro Leu Tyr Asn Ala Ser Gly Gly Ser Glu Asp Arg Leu Tyr
65                  70                  75                  80
Arg Asp Leu Glu Gly Ser Pro Ala Gln Pro Thr Lys His Ala Glu Gln
                85                  90                  95
Val Pro Tyr Leu Asn Glu Ile Ile Thr Thr Val Tyr Asn Gly Glu Arg
            100                 105                 110
Leu Gln Met Ala Arg Thr Arg Asn Leu Lys Asn Ala Val Val Ile Pro
        115                 120                 125
His Arg Asp Phe Val Glu Leu Asp Arg Glu Leu Asp Gln Tyr Phe Arg
    130                 135                 140
Thr His Leu Met Leu Glu Asp Ser Pro Leu Ala Phe His Ser Asp Asp
145                 150                 155                 160
Asp Thr Val Ile His Met Arg Ala Gly Glu Ile Trp Phe Leu Asp Ala
                165                 170                 175
Ala Ala Val His Ser Ala Val Asn Phe Ala Glu Phe Ser Arg Gln Ser
            180                 185                 190
Leu Cys Val Asp Leu Ala Phe Asp Gly Ala Phe Asp Glu Lys Glu Ala
        195                 200                 205
Phe Ala Asp Ala Thr Val Tyr Ala Pro Asn Leu Ser Pro Asp Val Arg
    210                 215                 220
Glu Arg Lys Pro Phe Thr Lys Glu Arg Glu Ala Gly Ile Leu Ala Leu
225                 230                 235                 240
Ser Gly Val Ile Gly Arg Glu Asn Phe Arg Asp Ile Leu Phe Leu Leu
                245                 250                 255
Ser Lys Val His Tyr Thr Tyr Asp Val His Pro Gly Glu Thr Phe Glu
            260                 265                 270
Trp Leu Val Ser Val Ser Lys Gly Ala Gly Asp Asp Lys Met Val Glu
        275                 280                 285
Lys Ala Glu Arg Ile Arg Asp Phe Ala Ile Gly Ala Arg Ala Leu Gly
    290                 295                 300
Glu Arg Phe Ser Leu Thr Thr Trp
305                 310
SEQUENCE NO. :16
   SEQUENCE LENGTH: 318
   SEQUENCE TYPE: amino acid
   TOPOLOGY: linear
   MOLECULE TYPE: protein
   ORIGINAL SOURCE:
      ORGANISM: Streptomyces sp.
      STRAIN: TH1
   SEQUENCE FEATURE:
      NAME/KEY: peptide
      location: 29 to 318
      IDENTIFICATION METHOD: S
   ORIGINAL SOURCE:
      ORGANISM: Escherichia coli
   IMMEDIATE SOURCE: pBluescriptIIKS+
   SEQUENCE FEATURE:
      NAME/KEY: peptide
      location: 1 to 27
      IDENTIFICATION METHOD: S
   SEQUENCE: 
Met Thr Met Ile Thr Pro Ser Ala Gln Leu Thr Leu Thr Lys Gly Asn
1               5                   10                  15
Lys Ser Trp Val Pro Gly Pro Pro Ser Arg Ser Arg Val Arg Ser His
            20                  25                  30
Ile Leu Gly Arg Ile Glu Leu Asp Gln Glu Arg Leu Gly Arg Asp Leu
        35                  40                  45
Glu Tyr Leu Ala Thr Val Pro Thr Val Glu Glu Glu Tyr Asp Glu Phe
    50                  55                  60
Ser Asn Gly Phe Trp Lys Asn Ile Pro Leu Tyr Asn Ala Ser Gly Gly
65                  70                  75                  80
Ser Glu Asp Arg Leu Tyr Arg Asp Leu Glu Gly Ser Pro Ala Gln Pro
                85                  90                  95
Thr Lys His Ala Glu Gln Val Pro Tyr Leu Asn Glu Ile Ile Thr Thr
            100                 105                 110
Val Tyr Asn Gly Glu Arg Leu Gln Met Ala Arg Thr Arg Asn Leu Lys
        115                 120                 125
Asn Ala Val Val Ile Pro His Arg Asp Phe Val Glu Leu Asp Arg Glu
    130                 135                 140
Leu Asp Gln Tyr Phe Arg Thr His Leu Met Leu Glu Asp Ser Pro Leu
145                 150                 155                 160
Ala Phe His Ser Asp Asp Asp Thr Val Ile His Met Arg Ala Gly Glu
                165                 170                 175
Ile Trp Phe Leu Asp Ala Ala Ala Val His Ser Ala Val Asn Phe Ala
            180                 185                 190
Glu Phe Ser Arg Gln Ser Leu Cys Val Asp Leu Ala Phe Asp Gly Ala
        195                 200                 205
Phe Asp Glu Lys Glu Ala Phe Ala Asp Ala Thr Val Tyr Ala Pro Asn
    210                 215                 220
Leu Ser Pro Asp Val Arg Glu Arg Lys Pro Phe Thr Lys Glu Arg Glu
225                 230                 235                 240
Ala Gly Ile Leu Ala Leu Ser Gly Val Ile Gly Arg Glu Asn Phe Arg
                245                 250                 255
Asp Ile Leu Phe Leu Leu Ser Lys Val His Tyr Thr Tyr Asp Val His
            260                 265                 270
Pro Gly Glu Thr Phe Glu Trp Leu Val Ser Val Ser Lys Gly Ala Gly
        275                 280                 285
Asp Asp Lys Met Val Glu Lys Ala Glu Arg Ile Arg Asp Phe Ala Ile
    290                 295                 300
Gly Ala Arg Ala Leu Gly Glu Arg Phe Ser Leu Thr Thr Trp
305                 310                 315
SEQUENCE NO. :17
   SEQUENCE LENGTH: 328
   SEQUENCE TYPE: amino acid
   TOPOLOGY: linear
   MOLECULE TYPE: protein
   ORIGINAL SOURCE:
      ORGANISM: Streptomyces sp.
      STRAIN: TH1
   SEQUENCE FEATURE:
      NAME/KEY: peptide
      location: 38 to 328
      IDENTIFICATION METHOD: S
   ORIGINAL SOURCE:
      ORGANISM: Escherichia coli
   IMMEDIATE SOURCE: pBluescriptIIKS+
   SEQUENCE FEATURE:
      NAME/KEY: peptide
      location: I to 37
      IDENTIFICATION METHOD: S
   SEQUENCE:

Met Thr Met Ile Thr Pro Ser Ala Gln Leu Thr Leu Thr Lys Gly Asn
1               5                   10                  15
Lys Ser Trp Val Pro Gly Pro Pro Ser Arg Ser Thr Val Ser Ile Ser
            20                  25                  30
Leu Ile Ser Asn Ser Arg Val Arg Ser His Ile Leu Gly Arg Ile Glu
        35                  40                  45
Leu Asp Gln Glu Arg Leu Gly Arg Asp Leu Glu Tyr Leu Ala Thr Val
    50                  55                  60
Pro Thr Val Glu Glu Glu Tyr Asp Glu Phe Ser Asn Gly Phe Trp Lys
65                  70                  75                  80
Asn Ile Pro Leu Tyr Asn Ala Ser Gly Gly Ser Glu Asp Arg Leu Tyr
                85                  90                  95
Arg Asp Leu Glu Gly Ser Pro Ala Gln Pro Thr Lys His Ala Glu Gln
            100                 105                 110
Val Pro Tyr Leu Asn Glu Ile Ile Thr Thr Val Tyr Asn Gly Glu Arg
        115                 120                 125
Leu Gln Met Ala Arg Thr Arg Asn Leu Lys Asn Ala Val Val Ile Pro
    130                 135                 140
His Arg Asp Phe Val Glu Leu Asp Arg Glu Leu Asp Gln Tyr Phe Arg
145                 150                 155                 160
Thr His Leu Met Leu Glu Asp Ser Pro Leu Ala Phe His Ser Asp Asp
                165                 170                 175
Asp Thr Val Ile His Met Arg Ala Gly Glu Ile Trp Phe Leu Asp Ala
            180                 185                 190
Ala Ala Val His Ser Ala Val Asn Phe Ala Glu Phe Ser Arg Gln Ser
        195                 200                 205
Leu Cys Val Asp Leu Ala Phe Asp Gly Ala Phe Asp Glu Lys Glu Ala
    210                 215                 220
Phe Ala Asp Ala Thr Val Tyr Ala Pro Asn Leu Ser Pro Asp Val Arg
225                 230                 235                 240
Glu Arg Lys Pro Phe Thr Lys Glu Arg Glu Ala Gly Ile Leu Ala Leu
                245                 250                 255
Ser Gly Val Ile Gly Arg Glu Asn Phe Arg Asp Ile Leu Phe Leu Leu
            260                 265                 270
Ser Lys Val His Tyr Thr Tyr Asp Val His Pro Gly Glu Thr Phe Glu
        275                 280                 285
Trp Leu Val Ser Val Ser Lys Gly Ala Gly Asp Asp Lys Met Val Glu
    290                 295                 300
Lys Ala Glu Arg Ile Arg Asp Phe Ala Ile Gly Ala Arg Ala Leu Gly
305                 310                 315                 320

Glu Arg Phe Ser Leu Thr Thr Trp
                325

## Claims

1. An isolated nucleic acid fragment coding for a protein having an amino acid sequence selected from Sequence Nos. 1, 2, 15, 16 and 17.

2. An isolated nucleic acid fragment selected from nucleic acid sequences of Sequence Nos. 3, 4, 13 and 14.

3. An isolated nucleic acid fragment, which hybridizes with the nucleic acid fragment according to claims 1 or 2, and codes for a protein having an enzymatic ability of hydroxylating the 3-position of L-proline and which acts on free L-proline in the presence of 2-ketoglutaric acid and divalent iron ions to produce cis-3-hydroxy-L-proline.

4. The nucleic acid fragment according to claims 1 to 3, wherein the gene is derived from microorganisms belonging to the genus Streptomyces or Bacillus.

5. The nucleic acid fragment according to claim 4, wherein the microorganisms are selected from those of Streptomyces canus ATCC12647, Streptomyces canus ATCC12646, Streptomyces sp. TH1 (FERM BP-4399), Bacillus sp. TH2 (FERM BP-4397) and Bacillus sp. TH3 (FERM BP-4398).

6. An expression vector constructed by inserting into a vector a nucleic acid fragment according to claims 1 to 5.

7. A transformed cell having an expression vector according to claim 6.

8. The transformed cell according to claim 7, which is selected from Escherichia coli XL2-Blue/pTH30 (FERM BP-5026) and Escherichia coli XL2-Blue/pTH75 (FERM BP-5409).

9. A protein having the amino acid sequence selected from Sequence Nos. 2, 15, 16 and 17.

10. A method for producing L-proline-3-hydroxylase, which comprises cultivating in a medium transformed cells according to claims 7 or 8. thereby producing and accumulating L-proline-3-hydroxylase, followed by collecting the L-proline-3-hydroxylase from the resulting culture.

11. The method for producing L-proline-3-hydroxylase according to claim 10, wherein L-proline is added to the medium.

12. A method for producing cis-3-hydroxy-L-proline, which comprises cultivating in a medium transformed cells according to claims 7 or 8, thereby producing and accumulating cis-3-hydroxy-L-proline, followed by collecting the cis-3-hydroxy-L-proline from the resulting culture.

13. The method for producing cis-3-hydroxy-L-proline according to claim 12, wherein the transformed cell has the ability of producing L-proline from the saccharide sources in the medium and accumulating cis-3-hydroxy-L-proline in the culture.

14. The method for producing cis-3-hydroxy-L-proline according to claim 12, wherein the transformed cell has the ability of producing 2-ketoglutaric acid from the saccharide sources in the medium and accumulating cis-3-hydroxy-L-prolin in the culture.

15. The method for producing cis-3-hydroxy-L-proline according to claim 12, wherein L-proline is added to the medium.

16. The method for producing cis-3-hydroxy-L-proline according to claim 12, wherein L-proline, 2-ketoglutaric acid and divalent iron ions are added to the medium.

17. A method for producing cis-3-hydroxy-L-proline, which comprises cultivating in a medium transformed cells according to claims 7 or 8, then converting L-proline into cis-3-hydroxy-L-proline in the presence of 2-ketoglutaric acid and divalent iron ions in the culture or in an aqueous medium while using the culture, the cells cultivated or a product to be prepared by processing the cells as the enzymatic source, followed by collecting the resulting cis-3-hydroxy-L-proline from the culture or the aqueous medium.

18. The method for producing cis-3-hydroxy-L-proline according to claim 17, wherein the transformed cell has the ability of producing L-proline from the saccharide sources in the medium and accumulating L-proline in the culture.

19. The method for producing cis-3-hydroxy-L-proline according to claim 17, wherein the transformed cell has the ability of producing 2-ketoglutaric acid from the saccharide sources in the medium and accumulating 2-ketoglutaric acid in the culture.

20. The method for producing cis-3-hydroxy-L-proline according to claim 17, wherein the product to be prepared by processing the cells cultivated is selected from dried the cells, lyophilized the cells, processing surfactant-treated cells, enzymatically-treated cells, ultrasonically-treated cells, mechanically-ground cells, mechanically-compressed cells, solvent-treated cells, fractionated cell proteins, immobilized cells, immobilized materials obtained by processing their cells, enzymes having the enzymatic ability of hydroxylating the 3-position of L-proline to be extracted from the cells, pure products to be prepared by purifying the enzymes and immobilized enzymes.

## Patentansprüche

1. Ein isoliertes Nukleinsäurefragment, das für ein Protein mit der Aminosäuresequenz ausgewählt aus den Sequenzen Nr. 1, 2, 15, 16 und 17 kodiert.

2. Isoliertes Nukleinsäurefragment, ausgewählt aus Nukleinsäuresequenzen der Sequenzen Nr. 3, 4, 13 und 14.

3. Isoliertes Nukleinsäurefragment, das mit dem Nukleinsäurefragment gemäß den Ansprüchen 1 oder 2 hybridisiert und für ein Protein kodiert, das die enzymatische Fähigkeit zur Hydroxylierung der 3-Position von L-Prolin besitzt, und das aus freiem L-Prolin in Anwesenheit von 2-Ketoglutarsäure und zweiwertigen Eisenionen cis-3-Hydroxy-L-Prolin herstellt.

4. Nukleinsäurefragment gemäß den Ansprüchen 1 bis 3, wobei das Gen aus Mikroorganismen der Gattung *Streptomyces* oder *Bacillus* stammt.

5. Nukleinsäurefragment gemäß Anspruch 4, wobei die Mikroorganismen ausgewählt sind aus *Streptomyces canus* ATCC12647, *Streptomyces canus* ATCC12646, *Streptomyces* sp. TH1 (FERM BP-4399), *Bacillus* sp. TH2 (FERM BP-4397) und *Bacillus* sp. TH3 (FERM BP-4398).

6. Expressionsvektor, hergestellt durch Insertion eines Nukleinsäurefragments gemäß den Ansprüchen 1 bis 5 in einen Vektor.

7. Transformierte Zelle, enthaltend einen Expressionsvektor gemäß Anspruch 6.

8. Transformierte Zelle gemäß Anspruch 7, die ausgewählt ist aus *Escherichia coli* XL2-Blue/pTH30 (FERM BP-5026) und *Escherichia coli* XL2-Blue/pTH75 (FERM BP-5409).

9. Protein mit der Aminosäuresequenz, ausgewählt aus den Sequenzen Nr. 2, 15, 16 und 17.

10. Verfahren zur Herstellung von L-Prolin-3-hydroxylase, umfassend das Kultivieren transformierter Zellen gemäß Anspruch 7 oder 8 in einem Medium, wodurch L-Prolin-3-hydroxylase hergestellt und angereichert wird, gefolgt von Gewinnen der L-Prolin-3-hydroxylase aus der resultierenden Kultur.

11. Verfahren zur Herstellung von L-Prolin-3-hydroxylase gemäß Anspruch 10, wobei L-Prolin dem Medium zugesetzt wird.

12. Verfahren zur Herstellung von cis-3-Hydroxy-L-Prolin, umfassend das Kultivieren transformierter Zellen gemäß den Ansprüchen 7 oder 8 in einem Medium, wodurch cis-3-Hydroxyl-L-Prolin hergestellt und angereichert wird, gefolgt von Gewinnen des cis-3-Hydroxy-L-Prolins aus der resultierenden Kultur.

13. Verfahren zur Herstellung von cis-3-Hydroxy-L-Prolin gemäß Anspruch 12, wobei die transformierfe Zelle die Fähigkeit zur Herstellung von L-Prolin aus Zuckerquellen in dem Medium besitzt und Anreichern von cis-3-Hydroxy-L-Prolin in der Kultur.

14. Verfahren zur Herstellung von cis-3-Hydroxy-L-Prolin gemäß Anspruch 12, wobei die transformierte Zelle die Fähigkeit zur Herstellung von 2-Ketoglutarsäure aus Zuckerquellen in dem Medium besitzt, und Anreichern von cis-3-Hydroxy-L-Prolin in der Kultur.

15. Verfahren zur Herstellung von cis-3-Hydroxy-L-Prolin gemäß Anspruch 12, wobei L-Prolin dem Medium zugesetzt wird.

16. Verfahren zur Herstellung von cis-3-Hydroxy-L-Prolin gemäß Anspruch 12, wobei L-Prolin, 2-Ketoglutarsäure und zweiwertige Eisenionen dem Medium zugesetzt werden.

17. Verfahren zur Herstellung von cis-3-Hydroxy-L-Prolin, umfassend Kultivieren transformierter Zellen gemäß Anspruch 7 oder 8 in einem Medium, dann Umsetzen von L-Prolin zu cis-3-Hydroxy-L-Prolin in Anwesenheit von 2-Ketoglutarsäure und zweiwertigen Eisenionen in der Kultur oder in einem wässrigen Medium, während die Kultur, die kultivierten Zellen oder ein Produkt, das durch Verarbeiten der Zellen hergestellt werden soll, als enzymatische Quelle verwendet wird, gefolgt von Gewinnen des resultierenden cis-3-Hydroxy-L-Prolins aus der Kultur oder dem wässrigen Medium.

18. Verfahren zur Herstellung von cis-3-Hydroxy-L-Prolin gemäß Anspruch 17, wobei die transformierte Zelle die Fähigkeit zur Herstellung von L-Prolin aus Zuckerquellen in dem Medium besitzt, und Anreichern von L-Prolin in der Kultur.

19. Verfahren zur Herstellung von cis-3-Hydroxy-L-Prolin gemäß Anspruch 17, wobei die transformierte Zelle die Fähigkeit zur Herstellung von 2-Ketoglutarsäure aus Zuckerquellen in dem Medium besitzt, und Anreichern von 2-Ketoglutarsäure in der Kultur.

20. Verfahren zur Herstellung von cis-3-Hydroxy-L-Prolin gemäß Anspruch 17, wobei das Prodükt, das hergestellt werden soll, durch Verarbeiten der kultivierten Zellen ausgewählt ist aus getrockneten Zellen, lyophilisierten Zellen, verarbeiteten Surfaktanzien-behandelten Zellen, enzymatisch behandelten Zellen, ultraschallbehandelten Zellen, mechanisch zermahlenen Zellen, mechanisch komprimierten Zellen, Lösungsmittel-behandelten Zellen, fraktionierten Zellproteinen, immobilisierten Zellen, immobilisierten Materialien erhalten durch Behandlung der Zellen, Enzymen mit der enzymatischen Fähigkeit zur Hydroxylierung der 3-Position von L-Prolin, das aus den Zellen extrahiert werden soll, reinen Produkten, die durch Reinigen der Enzyme und immobilisierten Enzyme hergestellt werden sollen.

## Revendications

1. Fragment d'acide nucléique isolé codant pour une protéine ayant une séquence d'acides aminés choisie parmi les Séquences Nos. 1, 2, 15, 16 et 17.

2. Fragment d'acide nucléique isolé choisi parmi les séquences d'acide nucléique de Séquence Nos. 3, 4, 13 et 14.

3. Fragment d'acide nucléique isolé, qui hybride avec le fragment d'acide nucléique selon les revendications 1 ou 2, et code pour une protéine ayant une aptitude enzymatique à hydroxyler la position 3 de la L-proline et qui agit sur la L-proline libre en présence d'acide 2-cétoglutarique et d'ions fer divalents pour produire la cis-3-hydroxy-L-proline.

4. Fragment d'acide nucléique selon les revendications 1 à 3, dans lequel le gène est dérivé de micro-organismes appartenant au genre Streptomyces ou Bacillus.

5. Fragment d'acide nucléique selon la revendication 4, dans lequel les micro-organismes sont choisis parmi ceux de Streptomyces canus ATCC12647, Streptomyces canus ATCC12646, Streptomyces sp. TH1 (FERM BP-4399), Bacillus sp. TH2 (FERM BP-4397) et Bacillus sp. TH3 (FERM BP-4398).

6. Vecteur d'expression construit par insertion dans un vecteur d'un fragment d'acide nucléique selon les revendications 1 à 5.

7. Cellule transformée ayant un vecteur d'expression selon la revendication 6.

8. Cellule transformée selon la revendication 7, qui est choisie parmi Escherichia coli XL2-Blue/pTH30 (FERM BP-5026) et Escherichia coli XL2-Blue/pTH75 (FERM BP-5409).

9. Protéine ayant la séquence d'acides aminés choisie parmi les Séquences Nos. 2, 15, 16 et 17.

10. Procédé pour la production de L-proline-3-hydroxylase, qui comprend la culture dans un milieu de cellules transformées selon les revendications 7 ou 8, produisant et accumulant ainsi la L-proline-3-hydroxylase, suivie par la collecte de la L-proline-3-hydroxylase à partir de la culture résultante.

11. Procédé pour la production de L-proline-3-hydroxylase selon la revendication 10, dans lequel de la L-proline est ajoutée au milieu.

12. Procédé pour la production de cis-3-hydroxy-L-proline, qui comprend la culture dans un milieu de cellules transformées selon les revendications 7 ou 8, produisant et accumulant ainsi la cis-3-hydroxy-L-proline, suivie par la collecte de la cis-3-hydroxy-L-proline à partir de la culture résultante.

13. Procédé pour la production de cis-3-hydroxy-L-proline selon la revendication 12, dans lequel la cellule transformée a l'aptitude à produire de la L-proline à partir des sources de saccharide dans le milieu et à accumuler la cis-3-hydroxy-L-proline dans la culture.

14. Procédé pour la production de cis-3-hydroxy-L-proline selon la revendication 12, dans lequel la cellule transformée a l'aptitude à produire de l'acide 2-cétoglutarique à partir des sources de saccharide dans le milieu et à accumuler la cis-3-hydroxy-L-proline dans la culture.

15. Procédé pour la production de cis-3-hydroxy-L-proline selon la revendication 12, dans lequel de la L-proline est ajoutée au milieu.

16. Procédé pour la production de cis-3-hydroxy-L-proline selon la revendication 12, dans lequel de la L-proline, de l'acide 2-cétoglutarique et des ions fer divalents sont ajoutés au milieu.

17. Procédé pour la production de cis-3-hydroxy-L-proline, qui comprend la culture dans un milieu de cellules transformées selon la revendication 7 ou 8, puis la conversion de la L-proline en cis-3-hydroxy-L-proline en présence d'acide 2-cétoglutarique et d'ions fer divalents dans la culture ou dans un milieu aqueux, tandis qu'on utilise la culture, les cellules cultivées ou un produit à préparer par traitement des cellules en tant que source enzymatique, suivie par la récolte de la cis-3-hydroxy-L-proline résultante à partir de la culture ou du milieu aqueux.

18. Procédé pour la production de cis-3-hydroxy-L-proline selon la revendication 17, dans lequel la cellule transformée a l'aptitude à produire de la L-proline à partir des sources de saccharide dans le milieu et à accumuler de la L-proline dans la culture.

19. Procédé pour la production de cis-3-hydroxy-L-proline selon la revendication 17, dans lequel la cellule transformée a l'aptitude à produire de l'acide 2-cétoglutarique à partir des sources de saccharide dans le milieu et à accumuler de l'acide 2-cétoglutarique dans la culture.

20. Procédé pour la production de cis-3-hydroxy-L-proline selon la revendication 17, dans lequel le produit à préparer par traitement des cellules cultivées est choisi parmi les cellules séchées, les cellules lyophilisées, les cellules traitées avec des agents tensio-actifs de traitement, les cellules traitées par voie enzymatique, les cellules traitées aux ultrasons, les cellules broyées mécaniquement, les cellules comprimées mécaniquement, les cellules traitées au solvant, les protéines de cellules fractionnées, les cellules immobilisées, les matières immobilisées obtenues par traitement de leurs cellules, les enzymes ayant l'aptitude enzymatique à hydroxyler la position 3 de la L-proline à extraire à partir des cellules, les produits purs à préparer par purification des enzymes, et les enzymes immobilisées.
